# EUROPÄISCHE PATENTSCHRIFT

(11) **EP 1 128 779 B1**
(45) Veröffentlichungstag und Bekanntmachung des Hinweises auf die Patenterteilung: **26.01.2005**
(21) Anmeldenummer: 99971688.9
(22) Anmeldetag: 03.11.1999
(51) Int. Cl.: A61C 9/00

(54) **DENTALER ABFORMLÖFFEL**
DENTAL IMPRESSION TRAY
PORTE-EMPREINTE DENTAIRE

(30) Priorität: 11.11.1998 DE 19852056
(43) Veröffentlichungstag der Anmeldung: 05.09.2001
(73) Patentinhaber: Dürr Dental GmbH & Co. KG, 74321 Bietigheim-Bissingen (DE)
(72) Erfinder: HAHN, Rainer, D-72074 Tübingen (DE)
(74) Vertreter: Ostertag, Reinhard
(86) Internationale Anmeldenummer: PCT/EP1999/008425
(87) Internationale Veröffentlichungsnummer: WO 2000/027302

(56) Entgegenhaltungen:
- WO-A-97/32536
- US-A- 1 517 197
- US-A- 2 963 786
- US-A- 5 370 533
- US-A- 5 722 832

## Beschreibung

Die Erfindung betrifft einen dentalen Abformlöffel gemäß dem Oberbegriff des Anspruches 1.

Bei klassischen Zahnabformungsverfahren wird ein Abformlöffel, der eine im wesentlichen U-förmige Formrinne aufweist, die durch eine Bodenwand und zwei in Umfangsrichtung verlaufende Wände begrenzt ist, noch außerhalb des Mundes des Patienten mit einem Abformmaterial gefüllt. Der Abformlöffel wird dann auf die abzuformende Zahnreihe aufgesetzt, wobei das Abformmaterial von den Zähnen verdrängt wird, so daß nach dem Aushärten des Abformmateriales ein Negativbild der Zahnreihe erhalten wird. Derart erhaltene Abformungen haben aber oft Fehler, die auf eingeschlossene Flüssigkeiten (Sulcus-Fluid, Blut, etc.) oder Luftblasen, mangelhafte Benetzung, Verzüge oder elastische Rückstellungen nach Verformung der plastischen Abformmasse sowie deren Aushärten unter Anpressdruck zurückzuführen sind.

Die US 5 370 533 A offenbart einen Abformlöffel, bei dem in der Bodenwand der Formrinne eine Mehrzahl von Öffnungen vorgesehen ist, die mit einem dahinter liegenden Verteilkanal für Abformmaterial in Verbindung stehen. Beim Arbeiten mit diesem Abformlöffel wird in den Abformlöffel zunächst pastöses Abformmaterial eingefüllt, und über dessen freie Oberfläche wird eine Trennfolie gelegt. Nach dem festen Aufdrücken des gefüllten Abformlöffels wird die Trennfolie abgenommen und durch das vorgeformte pastöse Abformmaterial werden mit einem Werkzeug Kanäle erzeugt, die zu den Öffnungen in der Bodenwand führen. Nach Wiederaufsetzen des Abformlöffels mit der mit Kanälen versehenen Vorform auf den Kiefer wird über die Öffnungen in der Bodenwand des Abformlöffels dünnflüssiges Abformmaterial in den schmalen Zwischenraum zwischen Gebissoberfläche und Vorform-Oberfläche gedrückt.

In der US 2 963 786 ist ein Abformlöffel beschrieben, der zur Verwendung mit pastösem Abformmaterial bestimmt ist. Am Boden einer Formrinne sind kleine Zapfen angeordnet, welche verhindern, daß die Spitzen der Zähne sich vollständig durch das Abformmaterial hindurcharbeiten, wenn der mit Abformmaterial gefüllte Abformlöffel fest auf den Kiefer gedrückt wird.

Die US 1 517 197 A offenbart einen Abformlöffel, bei dem das Innere mit Schwamm-Material ausgefüllt ist. Dieses dient einerseits dazu, Flüssigkeit aufzunehmen, andererseits dazu, die auf die Weichgewebe des Kiefers ausgeübte Kraft zu begrenzen.

In der US 5 722 832 A ist ein Abformlöffel offenbart, bei dessen Anwendung zunächst um die Zahnoberfläche mit einer Spritze flüssiges Abformmaterial gespritzt wird. Dieses ist blasenfrei und sorgt für eine saubere Abbildung. Nach diesem Aufspritzen des Präzisions-Abformmateriales wird der Abformlöffel mit einem Abformmaterial, welches Blasen enthält, auf den Kiefer aufgesetzt. Anstelle eines solchen Blasen aufweisenden Abformmateriales kann auch ein Schwamm verwendet werden.

Es wurde schon vorgeschlagen (WO 97/32536A), das Abformmaterial an den hinteren Enden des Abformlöffels her in den zwischen Abformlöffel und Kiefer begrenzten Formraum einzuspeisen und unter Unterdruckeinwirkung durch die Formrinne zum vorderen Ende derselben zu ziehen. Mit derartigen Abformlöffeln erhält man schon gute Zahnabdrücke, für manche Problemzonen (tiefe und enge Kavitäten, Kavitäteneinschnitte, Zahnfleischsäume usw.) wäre jedoch eine noch präzisere fehlerfreie Abformung wünschenswert.

Zur Lösung dieser Aufgabe wird durch die vorliegende Erfindung ein Abformlöffel mit den im Anspruch 1 angegebenen Merkmalen vorgeschlagen.

Bei dem erfindungsgemäßen Abformlöffel sind in der durch die Löffelwände begrenzten Formrinne Deflektorelemente angeordnet, welche das Abformmaterial so umlenken, daß dieses eine zur Achse der Zähne parallele Komponente der Strömungsgeschwindigkeit erhält. Dies begünstigt ein vollständiges Abformen hinterschnittener und tiefer Bereiche.

Vorteilhafte Weiterbildungen der Erfindung sind in den Unteransprüchen angegeben.

Deflektorelemente, wie sie im Anspruch 2 angegeben sind, kann man schon bei der Herstellung des Abformlöffels direkt an diesem anformen. Unterschiedliche Geometrien sind denkbar, insbesondere kuppenförmige, rampenförmige oder fischschuppenähnliche Geometrie der einzelnen Deflektorelemente.

Die Weiterbildung der Erfindung gemäß Anspruch 3 gestattet die Verwendung glattflächiger Grundteile, die sich besonders preisgünstig herstellen lassen. Dadurch, daß man die Deflektorelemente auf ein in das Grundteil einsetzbares Deflektionsteil legt, kann man auch die jeweils benötigte spezielle Strömungsumlenkung auf einfache Weise gewährleisten, ohne daß eine große Anzahl teurer mit Deflektorelementen versehener Grundteil bereitgehalten werden müßte.

Ein Deflektionsteil, wie es im Anspruch 4 angegeben ist, läßt sich besonders einfach durch Vakuumverformung einer Folie oder durch Spritzen herstellen.

Die Weiterbildung in der Erfindung gemäß Anspruch 5 und 6 sind im Hinblick auf eine möglichst gleichförmige Umlenkwirkung auf die verschiedenen Teilbereiche des Abformmaterialstromes von Vorteil.

Die Weiterbildung der Erfindung gemäß Anspruch 7 gestattet es, mit nur einem einzigen Deflektionsmaterial oder einer kleinen Anzahl unterschiedlicher Deflektionsmaterialien unterschiedliche Zahngrößen abzudecken, da dann, wenn die Deflektorelemehte sich zu weit in die Formrinne hineinerstrecken und in Berührung mit einem Zahn kommen, eine Verformung der entsprechenden Deflektionselemente erfolgt. Die verformten Deflektorelemente beeinträchtigen nicht die Güte des Abdruckes. Sie werden in verformtem Zustand in das Abformmaterial eingebaut, wobei auch dann, wenn die Deflektorlemente teilweise an der Oberfläche liegen, eine glatte Oberfläche erzielt wird, so daß nur eine optische, nicht aber eine funktionelle Unregelmäßigkeit vorliegt.

Deflektorelemente, wie sie im Anspruch 8 angegeben sind, zeichnen sich durch eine besonders einfache Geometrie und damit einfache Herstellbarkeit aus.

Die Weiterbildung der Erfindung gemäß Anspruch 9 gestattet ein Anordnen einer sehr großen Anzahl von Deflektorelementen auf kleinem Raum. Auch bei solchen Deflektorelementen läßt sich leicht sicherstellen, daß alle Teilbereiche des Abformmaterialstromes in gleicher Weise abgelenkt werden.

Die Weiterbildung der Erfindung gemäß Anspruch 10 gestattet auf besonders einfache Weise eine intensive Streuung der einzelnen Flüssigkeitsfäden in über den gesamten Querschnitt des Abformmaterialstromes hinweg gleichbleibender Weise. Offenporige Schaummaterialien sind im Handel als Plattenmaterial mit unterschiedlicher Porendichte (und damit Porengröße), unterschiedlicher Wandstärke und hergestellt aus unterschiedlichen Materialien erhältlich. Sie brauchen nur durch ein Schneidwerkzeug mit der jeweils benötigten Randkontur versehen zu werden.

In der vorliegenden Beschreibung und den Ansprüchen soll unter einem offenporigen Schaummaterial ein solches verstanden werden, bei welche benachbarte Poren strömungsmäßig in Verbindung stehen. Wie diese Verbindungen zwischen den einzelnen Poren entstehen, soll durch den Begriff offenporiges Schaummaterial nicht konkretisiert sein. Dieser Begriff soll insbesondere nicht auf solche Materialien beschränkt sein, die durch nachträgliches Zerstören von Porenwänden eines von Hause aus geschlossenporigen Materiales erhalten werden (z.B. durch Walzen oder sonstige mechanische Bearbeitung). Von den offenporigen Schaummaterialien stellen gemäß der hier verwendeten Terminologie die Skelettschäume eine spezielle Untermenge dar.

Schaummaterialien, wie sie im Anspruch 11 angegeben sind (Skelettschäume) , haben ein hohes Umlenkvermögen bei nicht zu großer Drosselung des Abformmaterialstromes.

Bevorzugte Porendichten bzw. Maschenweiten der offenporigen Schaummaterialien sind (ausgedrückt über die Porendichte) in den Ansprüchen 12 und 13 angegeben.

Die Ansprüche 14 und 15 geben Verhältnisse zwischen der Porengröße und der Wanddicke der offenporigen Schaumstoffmaterialien bzw. des Skelettschaummateriales an.

Die in Anspruch 16 bzw.17 angegebenen organischen Schaummaterialien und anorganischen Schaummaterialien werden bevorzugt, auch deshalb, weil gängige Abformmaterialien mit diesen Schaummaterialien einen guten Verbund eingehen können.

Die Weiterbildung der Erfingung gemäß Anspruch 18 gewährleistet eine hohe Ausbeute bei Herstellung der Schaummaterialschichten ausgehend von blockförmigen Zwischenprodukten.

Die Weiterbildung der Erfindung gemäß Anspruch 19 gestattet es, die Strömungsverhältnisse innehalb des Deflektionsteiles unterschiedlich vorzugeben.

Gemäß Anspruch 20 kann man einen Teil der Formrinne für das Abformmaterial sperren, so daß dieses insgesamt zwangsweise näher an den Zähnen fließt. Man erhält so auch eine Einsparung an teurem Abformmaterial. Durch die transversale Konturierung des flüssigkeitsundurchlässigen Bereiches des Deflektorteiles kann man dabei gewährleisten, daß der Abdruck ein Querschnittsprofil erhält, welches eine ausreichende mechanische Festigkeit des fertigen Abdruckes gewährleistet, z.B. einen U-Profil-Querschnitt.

Bei einem Abformlöffel gemäß Anspruch 21 kann man durch die Grundschicht eine Grobverteilung des Abformmaterials vornehmen, welche ohne große Drosselung des nicht umgelenkten Teiles des Abformmaterialstromes arbeitet, und die offenporige Oberschicht kann die im wesentlichen homogene Abgabe des Abformmateriales durch das Deflektionsteil sicherstellen, insbesondere, wenn die Grundschicht größere Poren aufweist als die von ihr getragene Schaummaterialschicht (Anspruch 22).

Die Weiterbildung der Erfindung gemäß Anspruch 23 erlaubt eine gute Abdichtung der Umfangswand des Grundteils gegew Mundgewebe.

Die Weiterbildung der Erfindung gemäß Anspruch 24 erlaubt auf einfache Weise ein Festlegen des Deflektionsteiles am Löffelgrundteil.

Die Weiterbildung der Erfindung gemäß Anspruch 25 findet dann bzw. insoweit Verwendung, als das Deflektionsteil die abzuformenden Zähne nicht berührt. Dadurch, daß das Abformmaterial die Deflektorelemente nicht benetzt, wird eine stärkere Umlenkung erhalten.

Berührt dagegen gemäß Anspruch 26 das Deflektionsteil (gegebenenfalls nur teilweise) die abzuformenden Zähne, so werden die Deflektionselemente insoweit als durch das Abformmaterial benetzbar ausgebildet. In diesem Falle kann man die Deflektorelemente zusätzlich zur Benetzung der Zahnoberflächen durch das Abformmaterial nutzbar machen. Zwischen den Deflektorelementen und dem Abformmaterial sowie den Zahnoberflächen bilden sich kleine Menisken aus, die die Benetzung der Zahnoberflächen durch das Abformmaterial begünstigen.

Die Weiterbildung der Erfindung gemäß Anspruch 27 gewährleistet, daß das Abformmaterial auf einfache Weise dem hinteren Abschnitt der Formrinne zugeführt wird.

Dabei läßt sich gemäß Anspruch 28 auf einfache Weise eine lösbare Verbindung zwischen Speiseteil und Löffelgrundteil gewährleisten.

Die Weiterbildung der Erfindung gemäß Anspruch 29 gestattet ein blasenfreies Abformen eines vollständigen Kiefers bis hin zum vorderen Ende.

Dabei ist mit der Weiterbildung der Erfindung gemäß Anspruch 30 sichergestellt, daß auch bei unsymetrischen Strömungsverhältnissen in den beiden Schenkeln des Abformlöffels Restluft bis zum vollständigen Füllen des Abformlöffels abgesaugt wird, der Abdruck somit durchgehend einschlußfrei ist.

Die Weiterbildung der Erfindung gemäß Anspruch 31 ist im Hinblick auf ein einfaches lösbares Verbinden des Vakuumanschlußteiles mit dem Löffelgrundteil von Vorteil. Dies ist insbesondere dann vorteilhaft, wenn das Vakuumanschlußteil ein einmal verwendetes Wegwerfteil ist. Die aufwendige Säuberung des Vakuumanschlußteiles für eine wiederverwendung kann dann entfallen.

Bei einem Abformlöffel gemäß Anspruch 32 läßt sich eine gute Abdichtung gegen Mundgewebe auch bei geringer mechanischer Belastung des Mundgewebes gewährleisten.

Eine besonders nachgiebige und unterschiedlichen lokalen Verhältnissen Rechnung tragende Abdichtung wird gemäß Anspruch 33 erhalten.

Dabei wird mit einer Doppel-Dichtlippenanordnung gemäß Anspruch 34 eine nochmals sicherere Abdichtung an Problemstellen gewährleistet.

Gemäß Anspruch 35 wird vorgeschlagen, den durch die Deflektorelemente gebildeten Strömungswiderstand und die Viskosität einer zusammen mit dem Abformlöffel verwendeten Abformmasse aufeinander abzustimmen. Auf diese Weise läßt sich gewährleisten, daß sich der zwischen Abformlöffel und Kiefer des Patienten eingeschlossene Formraum in der gewünschten Weise von hinten nach vorne und vom Boden des Abformlöffels zu den Zähnen hin füllt, so daß Einschlüsse von Luft und Flüssigkeiten vermieden werden. Bei dieser Abstimmung weiter einzuhaltende Randbedingung ist natürlich, daß das Abformmaterial ausreichend flüssig ist, um in enge hinterschnittene Bereiche der Zähne und deren Nachbarschaft eindringen zu können.

Anspruch 36 gibt eine bevorzugte Zuordnung zwischen der Viskosität des Abformmateriales und der Poren- bzw. Maschengröße des Deflektionsteiles.

Gerade im Zusammenhang mit einem Poren oder Maschen aufweisenden Deflektionsteil ist es besonders vorteilhaft, wenn das Abformmaterial gemäß Anspruch 37 nicht oder nur wenig thixotrop ist. Thixotrope Materialien würden sehr hohe Kräfte erfordern, um das Abformmaterial durch das Deflektionsteil zu bewegen. Ferner ist die Benetzung der Zahnoberfläche durch thixotrope Abformmaterialien weniger günstig.

Einen Abformlöffel kann man gemäß Anspruch 38 auch für therapeutische Zwecke einsetzen. Ein visköses Therapiematerial dient als Gefährt, mit welchem an die Wirksubstanzen für längere Zeit auch an tiefe Rücksprünge, in Zahnfleischtaschen, in okklusale Reliefs und ähnliche schlecht zugängliche Stellen des Kiefers eines Patienten bringen kann. Dabei kann das Gefährt für die medizinischen Wirkstoffe im übrigen weitgehend ähnliche Eigenschaften aufweisen wie ein Abformmaterial, so daß das Abformen von Zähnen und das Transportieren von Wirkstoffen zu den Zähnen und zum Zahnfleisch in einem Arbeitsgang erfolgen kann.

Bevorzugte Wirkstoffe sind in Anspruch 39 angegeben.

Die Erfindung wird nachstehend unter Bezugnahme auf die beiliegende Zeichnung näher erläutert. In dieser zeigen:
- Figur 1:: eine perspektivische Ansicht eines Abformlöffels für den Oberkiefers eines Patienten gesehen von hinten und oben;
- Figur 2:: eine perspektivische Ansicht des Abformlöffels nach Figur 1 gesehen von oben und vorne;
- Figur 3:: eine Aufsicht auf den Abformlöffel nach den Figuren 1 und 2;
- Figur 4:: einen Schnitt durch den Abformlöffel nach den Figuren 1 bis 3 längs der abgewinkelt-gekrümmten Schnittlinie IV-IV von Figur 3;
- Figur 5:: eine Aufsicht auf einen Ausschnitt eines Deflektorteiles, welches zusammen mit einem Abformlöffel nach Figuren 1 bis 4 verwendbar ist;
- Figur 6:: einen Schnitt durch das in Figur 5 gezeigte Deflektorelement längs der dortigen Schnittlinie VI-VI;
- Figur 7:: eine Aufsicht auf einen Abschnitt eines abgewandelten Deflektorelementes, das ebenfalls in Verbindung mit einem Abformlöffel nach den Figuren 1 bis 4 verwendbar ist;
- Figur 8:: einen Schnitt durch das in Figur 7 gezeigte Deflektorelement längs der dortigen Schnittlinie VIII-VIII;
- Figur 9:: einen transversalen Schnitt durch einen Abformlöffel, der ähnlich aufgebaut ist wie der Abformlöffel nach den Figuren 1 bis 4, jedoch zur Herstellung von Abdrücken des Unterkiefers dient, gezeigt unmittelbar nach der Positionierung auf dem Unterkiefer;
- Figur 10:: eine ähnliche Ansicht wie Figur 9, in welchem ein Zwischenzustand beim Füllen des zwischen Abformlöffel und Unterkiefer gebildeten Hohlraumes mit einem Abformmaterial wiedergegeben ist;
- Figur 11:: eine ähnliche Ansicht wie Figur 10, wobei jedoch nun der zwischen Abformlöffel und Unterkiefer liegende Raum vollständig mit Abformmaterial gefüllt ist;
- Figur 12:: einen transversalen Schnitt durch den Abformlöffel und den in diesen nach Abnehmen vom Unterkiefer verbleibenden Abdruck;
- Figur 13:: eine ähnliche Ansicht wie Figur 9, wobei jedoch ein Deflektorteil mit geringer Höhe verwendet wird am Beispiel eines Unterkieferabschnittes;
- Figur 14:: einen Längsschnitt durch einen ähnlichen Abformlöffel wie in Figur 13 gezeigt, jedoch nach Positionierung auf dem Oberkiefer eines Patienten;
- Figur 15:: eine ähnliche Ansicht wie Figur 14, wobei jedoch ein Deflektorteil, das im Abformlöffel angeordnet ist, zusätzlich mit einem Dichtabschnitt versehen ist;
- Figur 16:: eine ähnliche Ansicht wie Figur 15, wobei jedoch der Abformlöffel teilweise mit Abformmaterial gefüllt wiedergegeben ist;
- Figur 17:: eine ähnliche Ansicht wie Figur 16, wobei jedoch der Abformlöffel schon vor dem Positionieren auf dem Oberkiefer mit Abformmaterial gefüllt wurde;
- Figur 18:: einen Schnitt durch ein abgewandeltes VerbundDeflektionsteil, welcher senkrecht zur Hauptebene des Deflektionsteiles verläuft; und
- Figur 19:: eine ähnliche Ansicht wie Figur 18, wobei jedoch ein abgewandeltes Verbundmaterial gezeigt ist.

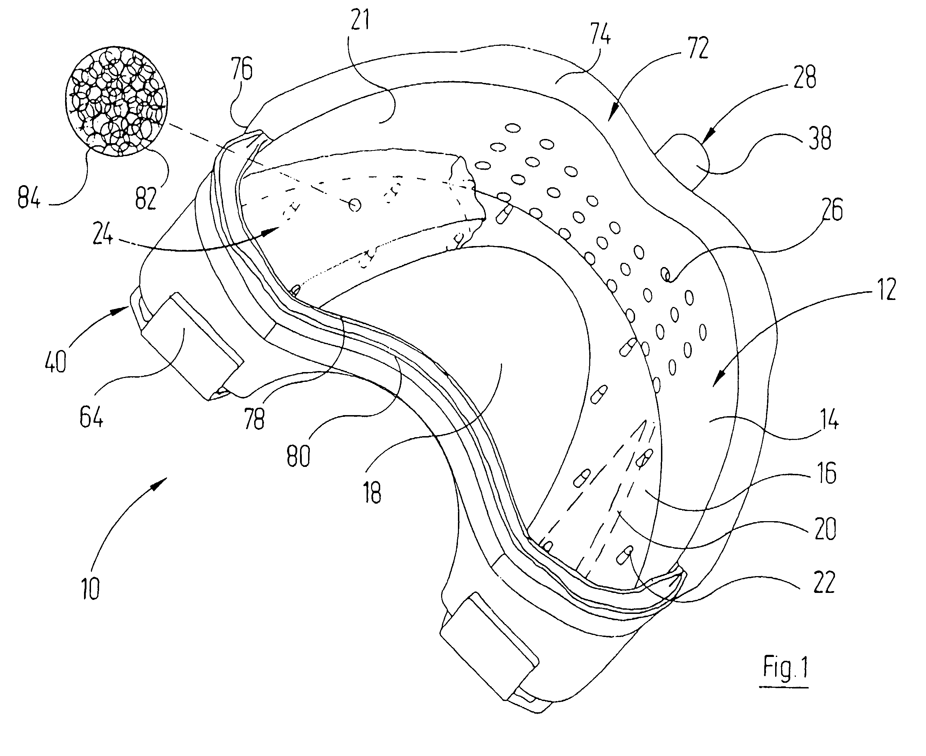

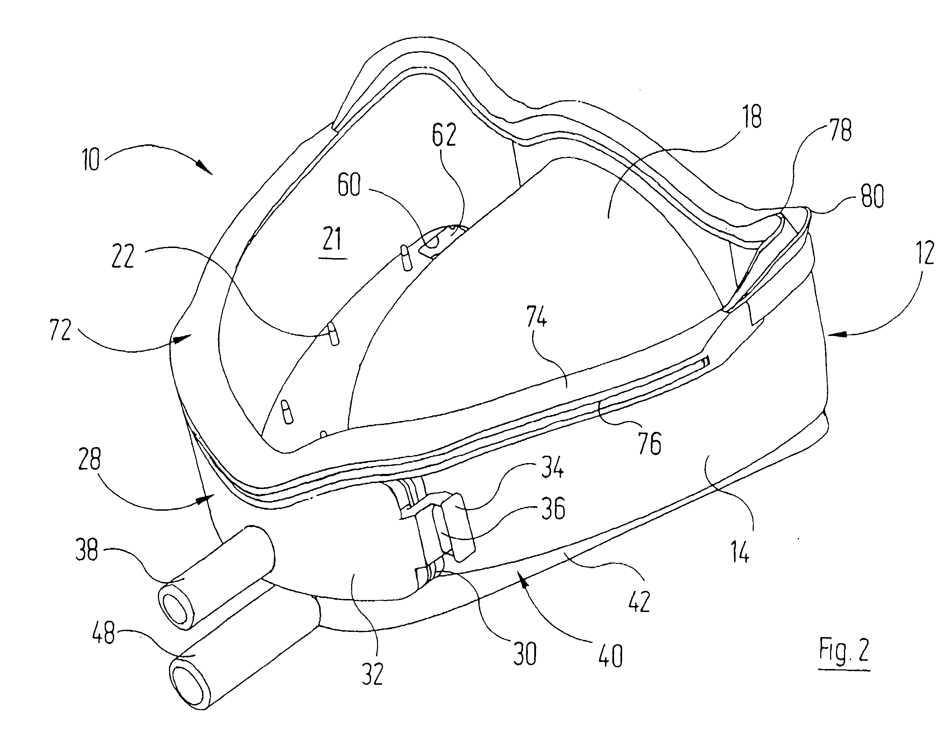

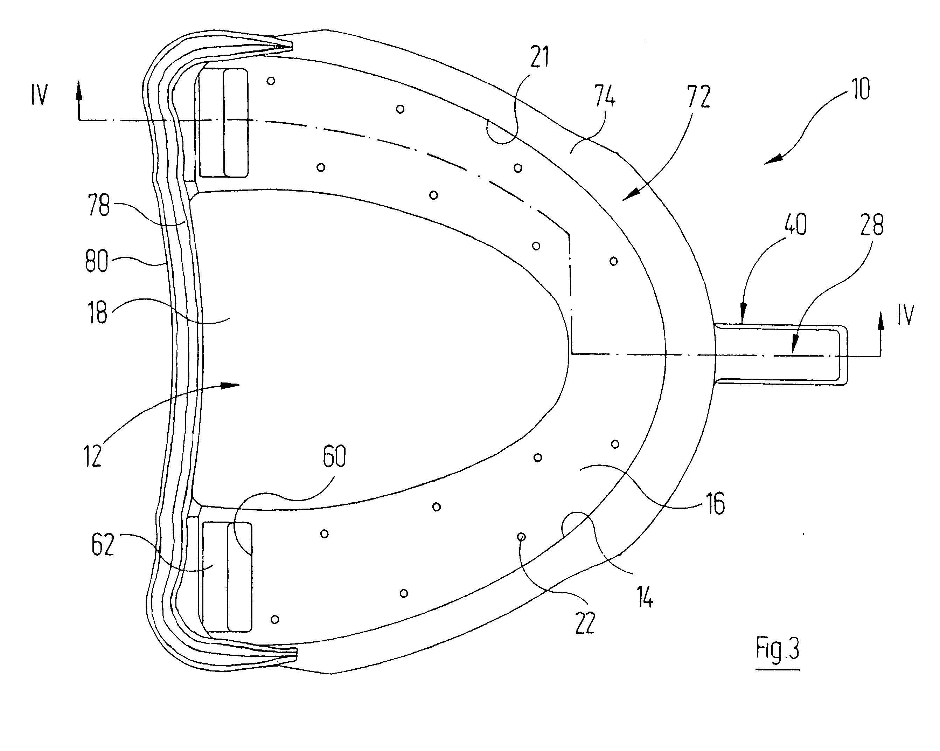

In den Figuren 1 bis 4 ist ein Abformlöffel insgesamt mit 10 bezeichnet. Er umfaßt ein insgesamt mit 12 bezeichnetes Grundteil, welches eine hufeisenähnlich gekrümmte Umfangswand 14 und eine ebenfalls hufeisenähnliche Ebene Bodenwand 16 umfaßt. Bei dem in den Figuren 1 bis 4 gezeigten Abformlöffel zur Verwendung an einem Oberkiefer ist an den inneren Rand der Bodenwand 16 eine Gaumenwand 18 angeformt, die grob gesprochen die Form eines Viertel-Ellipsoides hat. Bei einem Abformlöffel für einen Unterkiefer wäre anstelle der Gaumenwand 18 eine innere Umfangswand 20 vorgesehen, die in Figur 1 nur gestrichelt angedeutet ist. In beiden Fällen begrenzt das Grundteil 12 eine hufeisenförmige Formrinne 21.

Auf der Bodenwand 16 sind beim äußeren und inneren Rand und gegebenenfalls zusätzlich auch in der Mitte der Bodenwand 16 Haltezapfen 22 angeordnet, die zum Festlegen eines in Figur 1 nur gestrichelt angedeuteten Deflektorteiles 24 dienen.

In ihrem vorderen, proximalen Abschnitt ist die Umfangswand 14 mit einer Vielzahl von Vakuumöffnungen 26 versehen, die in einem regelmäßigen Muster angeordnet sind. Die Randkontur des mit Vakuumöffnungen 26 versehenen Bereiches der Umfangswand 14 ist im wesentlichen ein Rechteck.

Auf den vorderen Abschnitt der Umfangswand 14 ist ein insgesamt mit 28 bezeichnetes Vakuumanschlußteil unter Zwischenschaltung einer Flachdichtung 30 aufgeclipst. Das Vakuumanschlußteil 28 hat hierzu einen gemäß der Krümmung der Umfangswand 14 gekrümmten Basisabschnitt 32, an welchem seitlich zwei federnde Halteabschnitte 34 vorgesehen sind, die mit nicht näher gezeigten Rastmitteln versehen sind, die mit von der Vorderseite der Umfangswand 14 getragenen Befestigungszapfen 36 zur Bildung einer Rastverbindung zusammenarbeiten.

Auf seiner Außenseite trägt der Basisabschnitt 32 einen Anschlußstutzen 38, dessen Bohrung durch den Basisabschnitt 32 hindurchgeführt ist, so daß der Anschlußstutzen 38 über die Vakuumöffnungen 26 mit dem Inneren des Grundkörpers 26 in Verbindung steht.

Zwischen den Bohrungen auf der Außenseite des Abformlöffels und/oder der Inneseite des Vakuumanschlußteiles sind ggf. Fluidstrom-Bremselemente angeordnet, die ein vorzeitiges Verschließen von noch offenen Bohrungen in der Nachbarschaft bereits gefüllter Bohrungen verhindern. Solche Bremselemente können ebenfalls feinporige Strukturschaumelemente sein. Letztere können am Rand über das Vakuumanschlußteil geringfügig überstehen oder können teilweise mit einer geschlossenen Haut überzogen sein und so gleichzeitig durch das Zusammendrücken zwischen Vakuumanschlußteil und Löffelgrundteil Dichtfunktion übernehmen.

Auf die Unterseite der Bodenwand 16 ist ein Abformmaterial-Speiseteil 40 lösbar aufgesetzt. Dieses umfaßt eine senkrecht zur Hauptebene des Speiseteiles 40 verlaufende entsprechend der Umfangswand 14 gekrümmte Umfangswand 42 und eine zu dieser parallele innere Umfangswand 44 sowie eine Bodenwand 46.

Ein vorderer Abschnitt der Umfangswand 42 trägt einen Abformmaterial-Speisestutzen 48.

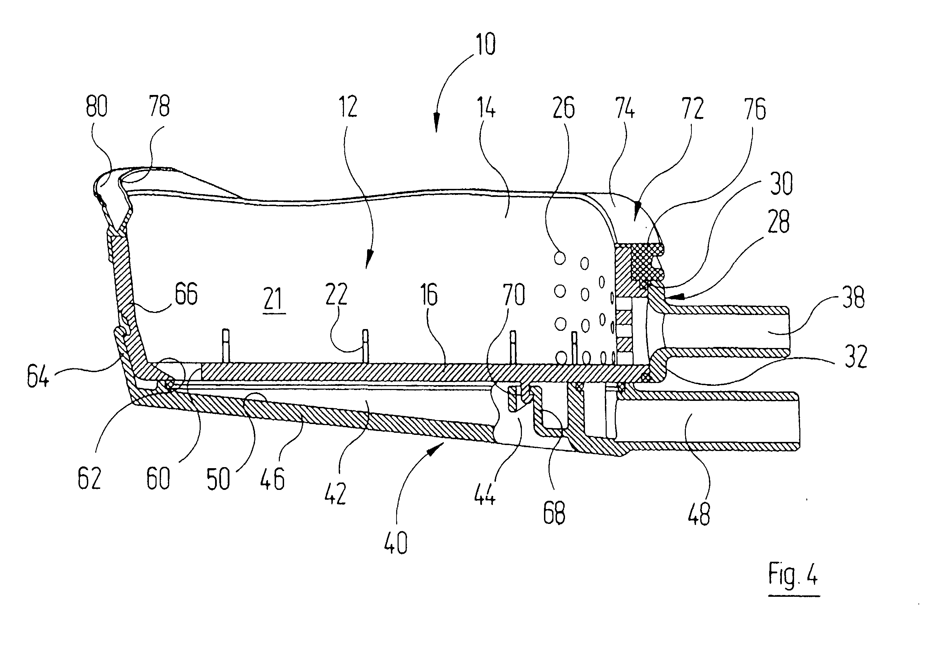

Wie aus Figur 4 ersichtlich, nähert sich die Bodenwand 46 mit zunehmendem Abstand vom Speisestutzen 48 der Bodenwand 16 des Grundteils 12 an. Die Bodenwand 16 des Grundteils 12 begrenzt so zusammen mit dem Speiseteil 40 einen Abformmaterial-Speisekanal 50, dessen Querschnitt zum hinteren Ende der beiden Schenkel des Speiseteiles 40 abnimmt. Die hinteren Enden der beiden Schenkel des hufeisenförmigen oder U-förmigen Speisekanales 50 stehen über bei den hinteren Enden der Bodenwand 16 dort vorgesehene in zur Strömungsrichtung transversaler Richtung breit Verbindungsöffnungen 60 mit dem Inneren des Grundteils 12 in Verbindung.

Wie aus Figur 4 ersichtlich, ist die eine Randwand 62 der Verbindungsöffnung 60 zum rückwärtigen Abschnitt der Umfangswand 16 hin abgeschrägt. Auf diese Weise kann Abformmaterial, welches dem Speisestutzen 48 zugeführt wird, ohne starke Richtungsänderung zum hinteren Ende des hufeisenförmigen Grundteils 12 geführt werden.

Zum lösbaren Verbinden des Speiseteiles 40 mit dem Grundteil 12 haben die hinteren Enden des Speiseteiles jeweils einen Rastarm 64, der mit einer passenden Rastvertiefung 66 im hinteren Abschnitt der Umfangswand 14 zusammenarbeitet. Zum Fixieren des vorderen Abschnittes des Speiseteiles 40 dient ein von der Umfangswand 40 getragener Rastarm 68, der mit einem hierzu passenden Rastarm 70 zusammenarbeitet, der von der Unterseite der Bodenwand 16 getragen ist.

Auf den oberen Rand der Umfangswand 14 ist ein insgesamt mit 72 bezeichnetes Dichtungsteil aus elastomerem Material angeordnet. Letzteres hat einen oberen horizontalen Profilabschnitt 74, der die Oberseite der Umfangswand 14 übergreift und einen die Außenseite des oberen Endes der Umfangswand 14 übergreifenden zweiten Profilabschnitt 76 mit U-förmigem Querschnitt.

Im hinteren Abschnitt des Dichtungsteiles 72 sind an den ersten Profilabschnitt 74 zwei Dichtlippen 78, 80 angeformt, die V-förmig angestellt sind (Öffnungswinkel des V etwa 50°). Die Dichtlippen 78, 80 dienen zum Abdichten des Abformlöffels 10 gegen das Gaumensegel.

Das Deflektorteil 24 hat Randkonturen, die denen der Bodenwand 16 entsprechen. Mit seinen hinteren Abschnitten überdeckt das Deflektorteil 24 die Verbindungsöffnungen 60.

Das Deflektorteil 24 ist aus einem Skelettschaummaterial hergestellt. Wie aus der Ausschnittvergrößerung von Figur 1 ersichtlich, hat ein solches Material ähnliche Struktur wie ein dreidimensionales Gewirk. Bei näherer Betrachtung erkennt man einzelne fadenähnliche Skelettzweige 82, die so geformt sind, daß sie Maschen oder Poren 84 bilden. In der Praxis wird ein derartiges Skelettschaummaterial dadurch erhalten, daß man ein Polyurethan-Schaummaterial retikuliert und kontrolliert karbonisiert. Die Porenwände dieses Schaummateriales ziehen sich dann auf die fadenähnlichen Skelettzweige 82 zusammen.

Ein derartiges Skelettschaummaterial stellt für ein flüssiges Material makroskopisch gesehen einen Strömungswiderstand dar, dessen Größe über die Größe der Maschen bzw. Poren 84 und die Dicke der fadenähnlichen Skelettzweige 82 eingestellt werden kann. Darüber hinaus stellen die verschiedenen fadenähnlichen Skelettzweige 82 mikroskopisch gesehen Streustellen für den Flüssigkeitsfluß dar, welche einen ursprünglich in einer ersten Richtung verlaufenden Flüssigkeitsstrom in unterschiedliche Richtungen ablenken können.

Grob gesprochen wird ein Abformlöffel, wie er obenstehend anhand der Figuren 1 bis 4 erläutert wurde, folgendermaßen eingesetzt (Einzelheiten werden weiter unten genauer beschrieben):

Das Vakuumanschlußteil 28 wird mit einer Vakuumquelle verbunden, das Speiseteil 40 mit einer Quelle für flüssiges Abformmaterial. Zunächst bleibt die Strömungsverbindung zwischen dem Speiseceil 40 und der Abformmaterialquelle geschlossen.

Nach dem Aufsetzen des Abformlöffels 10 auf den Oberkiefer saugt sich der Abformlöffel 10 am Zahnfleisch und der Gaumenplatte fest. Nach Öffnen der Strömungsverbindung zwischen dem Speiseteil 40 und der Abformmaterialquelle fließt flüssiges Abformmaterial zu den hinten im Grundteil 12 vorgesehenen Verbindungsöffnungen 60.

Dort tritt das flüssige Abformmaterial in das Deflektorteil 24 ein. Ein Teil des Abformmateriales läuft im Deflektorteil 24 zunächst parallel zur Bodenwand 16 weiter, Teile des parallel zur Bodenwand fließenden Stromes an Abformmaterial werden aber durch die fadenähnlichen Skelettzweig 82 umgelenkt und erhalten eine senkrecht zur Bodenwand 16 verlaufende Bewegungskomponente.

Der zwischen dem Oberkiefer und dem Abformlöffel 10 befindliche Formraum wird somit zunehmend von hinten mit Abformmaterial gefüllt, wobei ständig Luft aus dem Abformlöffel 10 über das Vakuumanschlußteil 28 angesaugt wird, bis der ganze Formraum blasenfrei ausgefüllt ist.

Das Vakuumanschlußteil kann auch ohne Anschluß an einer Vakuumpumpe betrieben werden. In diesem Falle dient es nur zur Entlüftung.

Erreicht das Abformmaterial den Anschlußstutzen 38, so wird der Füllvorgang beendet. Dies kann automatisch geschehen, indem man in oder hinter dem Anschlußstutzen 38 einen Filterstopfen vorsieht, der zwar für Luft, nicht jedoch für das höhere Viskosität aufweisende Abformmaterial durchlässig ist.

Die senkrecht zur Bodenwand 16 gerichtete Bewegungskomponente des flüssigen Abformmateriales ist wichtig, um auch in den hinterschnittenen Zwischenräumen zwischen aufeinanderfolgenden Zähnen und im Übergangsbereich zwischen Zahnkrone und Zahnhals, also beim Zahnfleischrand eine saubere und präzise Abformung zu erhalten.

Verwendet man als Material für das Deflektionsteil 24 ein Skelettschaummaterial, so hat man eine sehr große Anzahl im einzelnen unregelmäßig verteilter fadenähnlicher Skelettzweige 82, so daß man insgesamt auch eine im Mittel gleichförmige "Streuung" des Abformmateriales in die unterschiedlichen Raumwinkel erhält. Das Skelettschaummaterial wirkt somit für das Abformmaterial ähnlich wie eine Mattscheibe für Licht. Auf diese Weise sind Schatten im erhaltenen Abdruck vermieden.

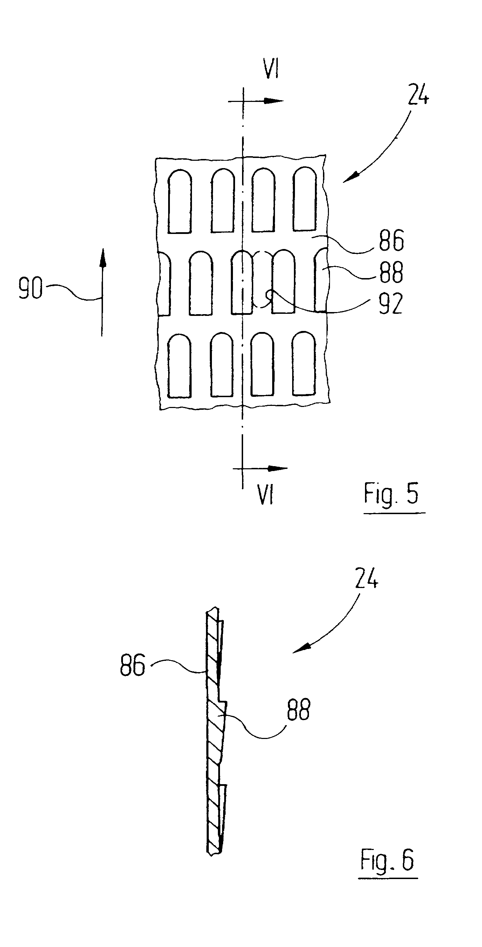

Anstelle des oben beschriebenen Deflektorteiles aus Skelettschaummaterial kann man auch ein Deflektorteil 24 verwenden, wie es teilweise in den Figuren 5 und 6 gezeigt ist. Ein solches Deflektorteil kann auch zusammen mit einem zusätzlichen anderen Deflektorteil, z.B. einem Skelettschaum, verwendet werden.

Eine Grundwand 86 trägt eine Vielzahl von schuppenähnlichen Deflektorrampen 88, die beim hier gezeigten Ausführungsbeispiel in Reihen angeordnet sind. Dabei sind die Deflektorrampen 88 benachbarter Reihen jeweils um eine halbe Teilung versetzt. Die Höhe der Deflektorrampen 88 kann, falls gewünscht, in Strömungsrichtung (Pfeil 90) variieren, z.B. mit abnehmendem Abstand vom Vakuumanschlußteil 28 zunehmend, um dort eine verstärkte Ablenkwirkung zu erhalten. Die Grundwand 86 kann in den von Deflektorrampen 88 freien Bereichen zusätzlich mit Durchbrechungen versehen sein, wie gestrichelt bei 92 angedeutet, die weitere Streuzentren für Abformmaterial bilden.

Fertigt man das in den Figuren 5 und 6 gezeigte Deflektorteil aus einem weichen Kunststoffmaterial oder aus Wachs, so können die Deflektorrampen 88 auch verhältnismäßig hoch sein, da sie gegebenenfalls durch die Spitzen eines Zahnes leicht verformt werden können.

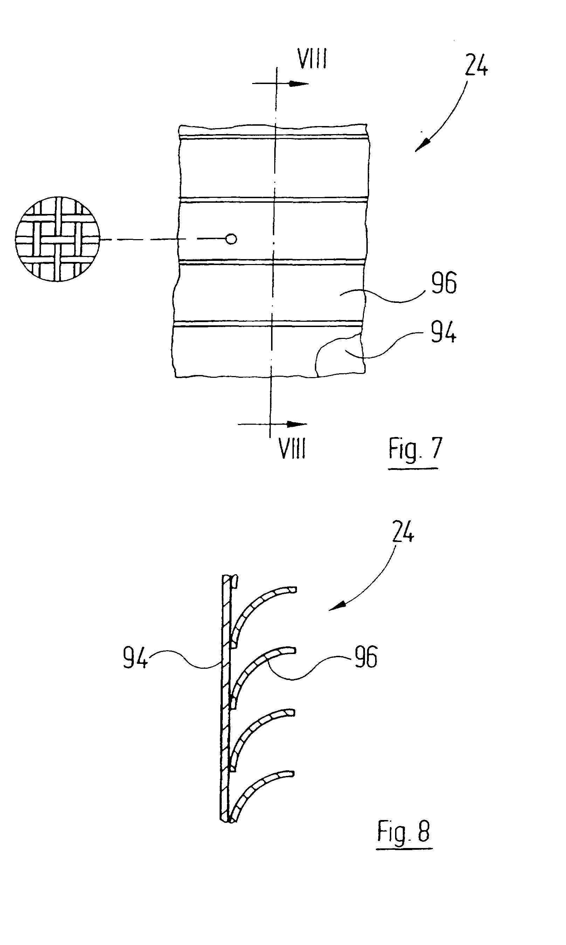

Das weiter abgewandelte Deflektorelementmaterial, welches in den Figuren 7 und 8 gezeigt ist, hat eine Grundwand 94, die gekrümmte Deflektor-Drahtnetze 96 trägt. Die Drahtnetze 96 lassen jeweils einen Teil des ankommenden Abformmateriales hindurchtreten und lenken einen anderen Teil dieses Materiales in Richtung auf die Zähne zu ab. Über die Maschenweite und den Durchmesser der einzelnen Drähte läßt sich der Anteil des umgelenkten Abformmateriales vorgeben. Bei einem solchen Deflektorelementmaterial kann man das Ausmaß der Umlenkung in Abhängigkeit vom Abstand der Umlenkstelle vom Vakuumanschlußteil 28 dadurch variieren, daß man Drahtnetze unterschiedlicher Maschenbreite und/oder unterschiedlichen Drahtdurchmessers und/oder unterschiedlicher Steigung und/oder Krümmung verwendet.

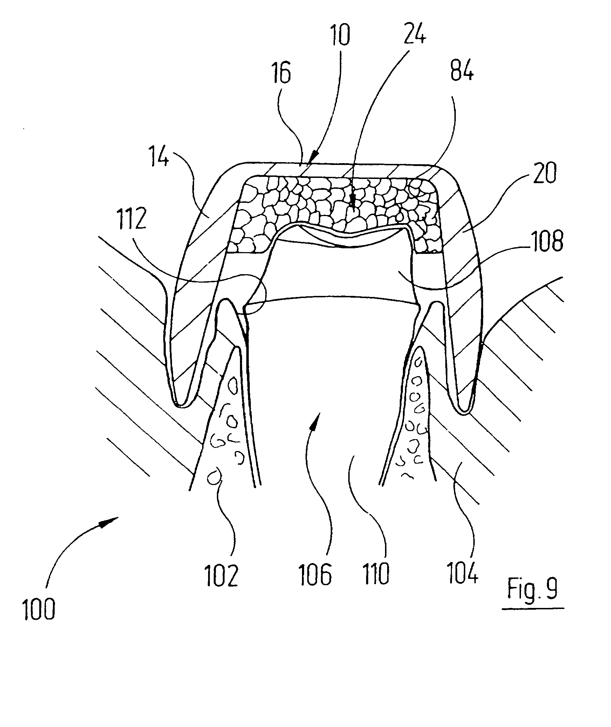

Anhand der Figuren 9 bis 11 wird nun die Anwendung eines Abformlöffels, wie er oben beschrieben wurde, in Verbindung mit einem "hohen" Deflektorteil beschrieben. Unter einem "hohen" Deflektorteil soll ein solches verstanden werden, welches beim Aufsetzen des Abformlöffels auf den Kiefer durch mindestens einen Zahn verformt wird. Unter einem "niederen" Deflektorteil soll dagegen ein solches verstanden werden, dessen Oberseite nach Positionierung des Abformlöffels auf dem Kiefer an allen Stellen Abstand zu den Zähnen aufweist. Bei einem typischen "niederen" Deflektorteil darf aber der Abstand zwischen der Oberseite des Deflektoreteils und den Zähnen nicht sehr groß sein. In der Praxis sollte dieser Abstand 1 bis 2 mm nicht überschreiten.

In Figur 9 ist ein Teil eines insgesamt mit 100 bezeichneten Unterkiefers wiedergegeben. Man erkennt bei 102 den Kieferknochen, bei 104 das Zahnfleisch. Ein Zahn ist insgesamt mit 106 bezeichnet, er hat eine Krone 108 und eine Wurzel 110. Durch den oberen Bereich der Zahnwurzel 110 und das Zahnfleisch 104 ist eine Zahnfleischtasche 112 begrenzt, die sich nach oben hin keilförmig erweitert. Gerade im Übergangsbereich zwischen der Krone 108 und dem Zahnhals ist eine präzise Abformung notwendig, wenn anhand der Abformung Kronen oder Brücken hergestellt werden sollen. Ansonsten ergibt sich gerade in diesem kritischen Bereich eine schlechte Formanpassung des Restaurationsteiles an den präparierten Zahn mit der Konsequenz, daß das Restaurationsteil schlecht sitzt und durch Spalte zwischen dem Restaurationsteil und der präparierten Zahnoberfläche Bakterien eindringen können.

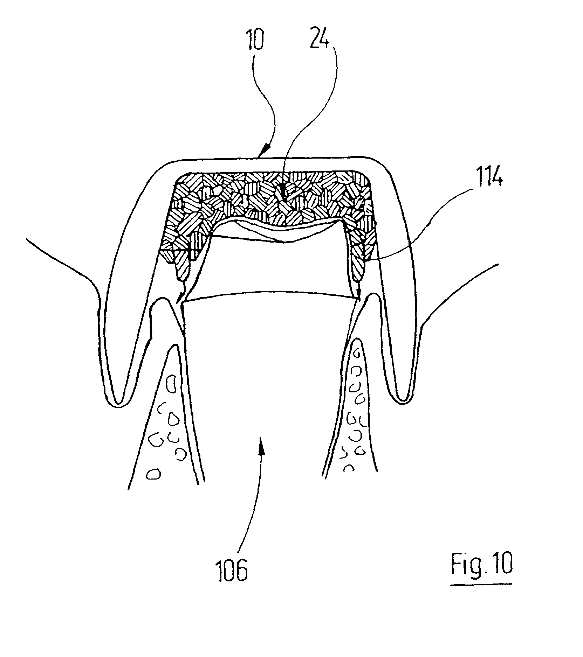

In Figur 10 ist die Situation wiedergegeben, in welcher das Innere des Abformlöffels teilweise mit Abformmaterial 114 gefüllt ist. Durch die schon oben angesprochene Streuung des Abformmateriales an dem aus Skelettschaum hergestellten Deflektionsteil 24 erhält man im Inneren des Deflektionsteiles 24 unterschiedlich gerichtete Teilströme, was durch die Lage einer Schraffur innerhalb der einzelnen Maschen/Poren 84 angedeutet ist. Dieses Andeuten ist zur Verdeutlichung übertrieben dargestellt, da der Fluß des Abformmateriales eine gleichbleibende Grundkomponente aufweist, die von den Verbindungsöffnungen 60 zum Vakuumanschlußteil 28 hin gerichtet ist.

Aus Figur 10 ist ferner ersichtlich, daß Teile des flüssigen Abformmateriales schon die Unterseite des Deflektorteiles 24 verlassen haben und sich im Formhohlraum nach unten bewegen.

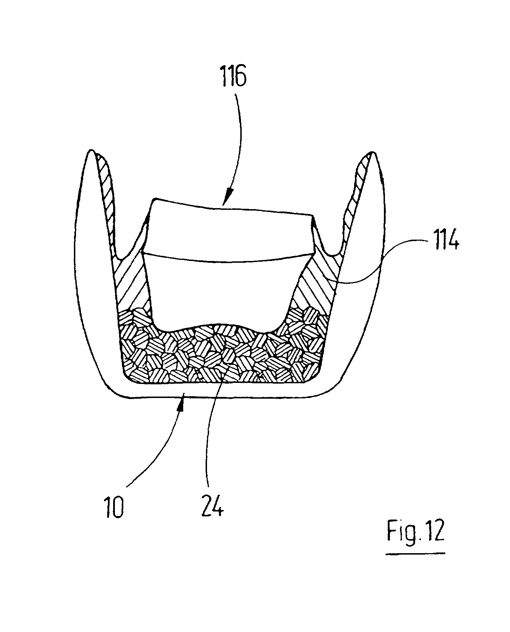

Figur 12 zeigt den Zustand nach vollständigem Ausfüllen des zwischen Abformlöffel 10 und Unterkiefer 100 begrenzten Raumes mit Abformmaterial 114.

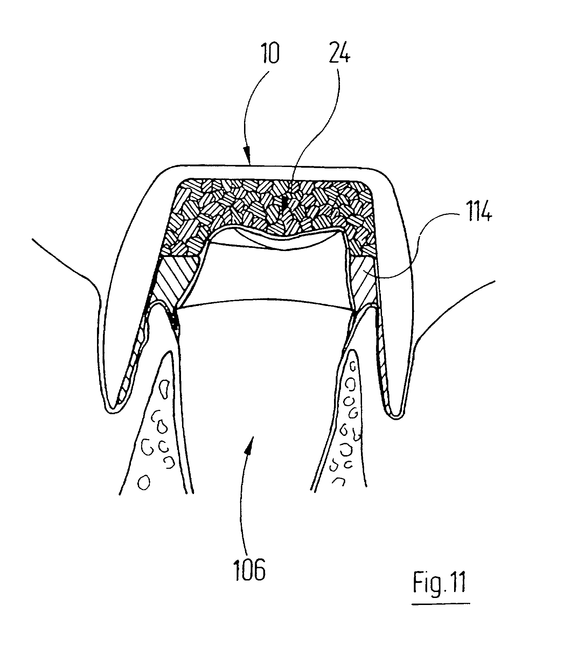

Figur 11 zeigt den Abformlöffel 10 mit fertigem Abdruck 116 nach Abnehmen des Abformlöffels 10 vom Unterkiefer 100 (und Umdrehen des Abformlöffels).

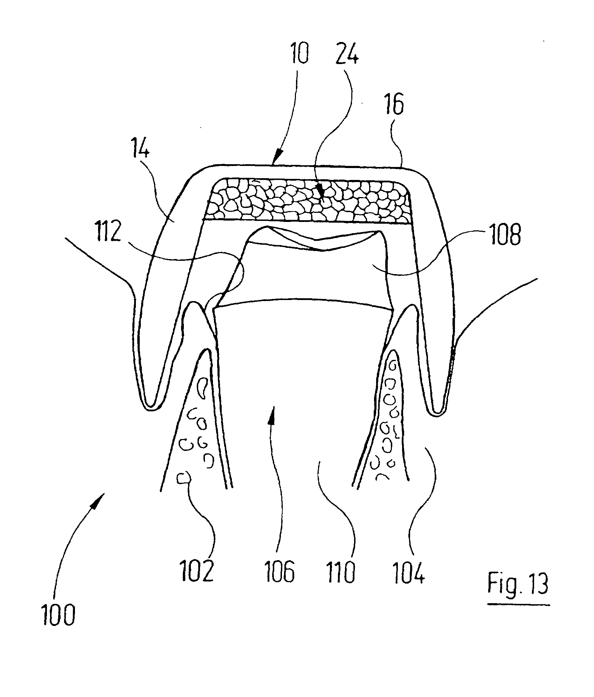

Figur 13 zeigt einen Abformlöffel 10, in welchen ein niederes Deflektorteil 24 eingesetzt ist.

Das Füllen des zwischen dem Abformlöffel 10 und einem Oberkiefer 118 begrenzten Raumes mit Abformmaterial erfolgt ähnlich wie vorstehend für die Figuren 9 bis 11 beschrieben, mit der Maßgabe, daß das Deflektorteil 24 von den Endflächen der Zähne überall beabstandet ist, so daß sich die unterschiedlichen Geschwindigkeitskomponenten der aus den Maschen bzw. Poren 84 austretenden Teilströme etwas ausgeglichen haben, bevor das Material auf die Zähne trifft, und deshalb die Geschwindigkeits-Zusatzkomponente für die verschiedenen Teilstrahlen im wesentlichen parallel zur Längsachse des Zahnes 106 gerichtet ist.

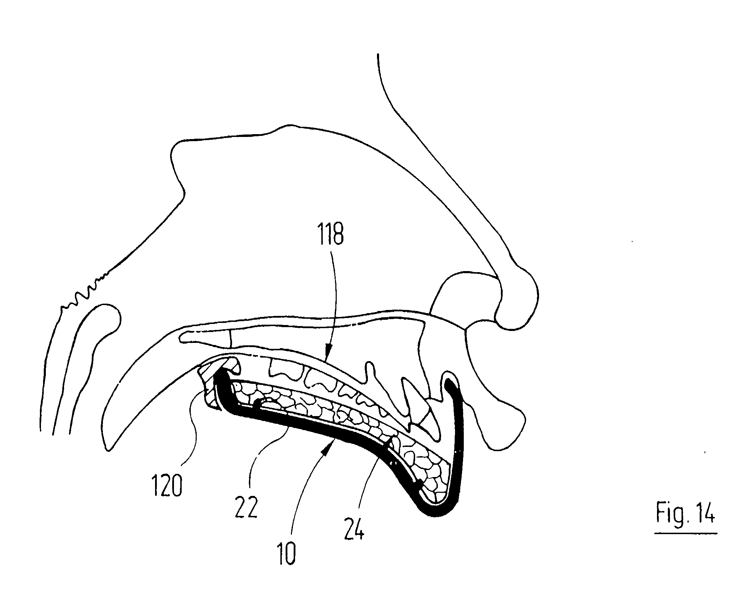

Figur 14 zeigt einen abgewandelten Abformlöffel 10 zur Abformung eines Oberkiefers. Auf der Umfangswand 14 ist das Dichtungsteil 72 weggelassen. Das obere Ende der Umfangswand 14 ist ballig ausgebildet, so daß es direkt mit dem Zahnfleisch zusammenarbeiten kann. Zur Abdichtung des hinteren Endes des Abformlöffels 10 gegen das Gaumensegel ist ein plastisch verformbares Dichtmaterial 120 vorgesehen, das auf den hinteren Abschnitt der Umfangswand 14 vor Applikation des Abformlöffels 10 aufgetragen wurde.

Wiederum wird ein niederes Deflektorteil 24 verwendet, wobei dieses in Proximal/Distal-Richtung so konturiert ist, daß die freie Oberfläche des Deflektorelementes 24 im wesentlichen konstanten Abstand von den Zahnspitzen aufweist.

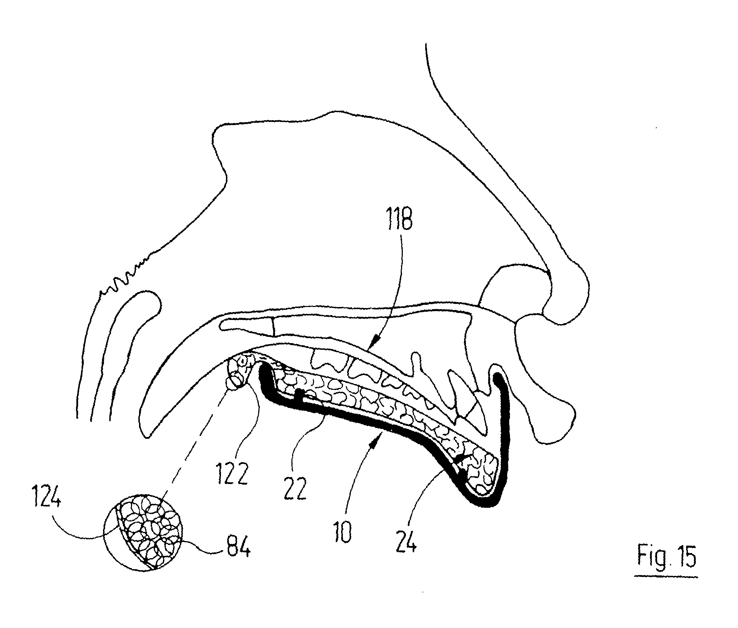

In Abwandlung des Ausführungsbeispiels nach Figur 14 weist das Deflektorteil 24 nach Figur 15 einen angeformten Dichtabschnitt 122 auf. Wie in einer Ausschnittsdarstellung wiedergegeben ist der Dichtabschnitt 122 durch eine aufgebrachte Haut 124 für Flüssigkeit undurchlässig gemacht, so daß das Material des Deflektorteiles 24 zugleich als nachgiebiges undurchlässiges Dichtmaterial dienen kann.

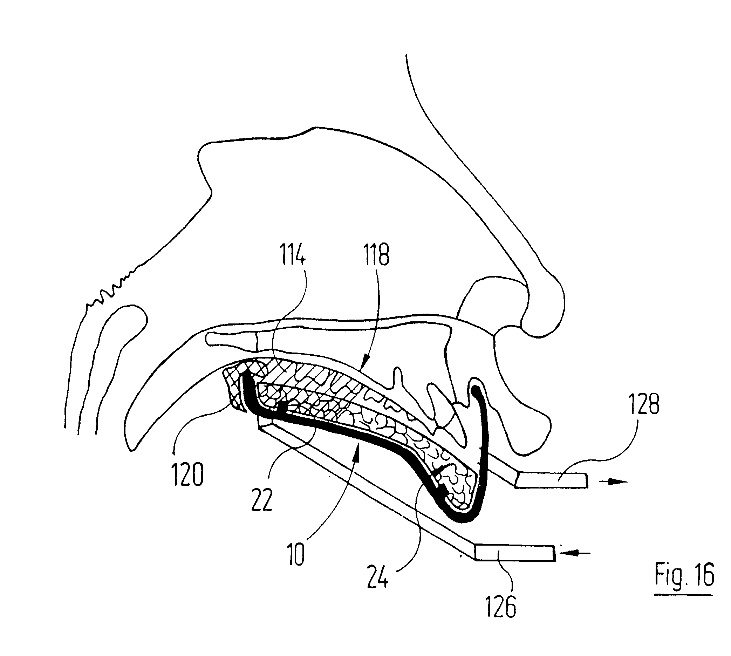

Figur 16 zeigt das Ausführungsbeispiel nach Figur 14 in einem Zwischenzustand der Füllung des zwischen Abformlöffel 10 und Oberkiefer 118 begrenzten Formraumes. Die Schenkel des Abformlöffels 10 sind von hinten her schon teilweise durch Abformmaterial 114 gefüllt, wie durch eine Schraffur angedeutet. Dabei soll die Richtung der Schraffur zugleich die Richtung derjenigen Material-Teilströme wiedergeben, die aus der Oberseite des Deflektorteiles 24 austreten. Man erkennt die von hinten nach vorn gerichtete Grundkomponente und die hierzu senkrechte zu den Zähnen hin verlaufende Komponente.

Die Zuführung von Abformmaterial zu den Verbindungsöffnungen 60 erfolgt durch direkt zu den Verbindungsöffnungen 60 verlaufende Schläuche 126, deren zweite Enden mit der Abformmaterialquelle verbunden sind, und die Vakuumbeaufschlagung des vorderen Abschnittes des Abformlöffels 10 erfolgt durch einen weiteren Schlauch 128, der mit der Vakuumquelle verbunden ist.

In Abwandlung kann der Schlauch 128 auch einfach mit der Atmosphäre kommunizieren. Er dient dann nur der Entlüftung des durch den Abformlöffel 10 und den Kiefer des Patienten begrenzten Formraumes.

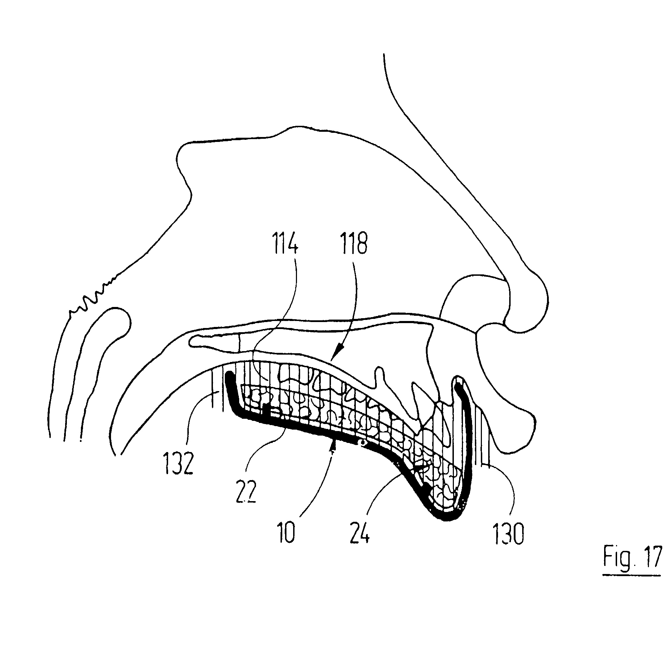

Gemäß Figur 17 kann man auch so vorgehen, daß man den mit dem Deflektorteil 24 versehenen Abformlöffel 10 schon vor dem Aufsetzen auf den Oberkiefer mit Abformmaterial 114 füllt. Beim Aufsetzen auf den Oberkiefer wird ein Teil des im Abformlöffel 10 befindlichen Abformmateriales nach außen verdrängt, wie bei 130 bzw. 132 angedeutet.

Auch in diesem Falle ist das Deflektorteil 24 nützlich, da es dafür sorgt, daß ein löffelinternes Verlagern von Abformmaterial nicht einfach parallel zur Bodenwand 16 des Abformlöffels erfolgt, vielmehr hierdurch zur Bodenwand 16 senkrechte Bewegungskomponenten erzeugt werden, wodurch ein sauberes Abformen auch von tiefen Einschnitten von Kavitäten und im Übergangsbereich zwischen Zahnkrone und Zahnwurzel erhalten wird, genauso wie bei den oben beschriebenen Ausführungsformen.

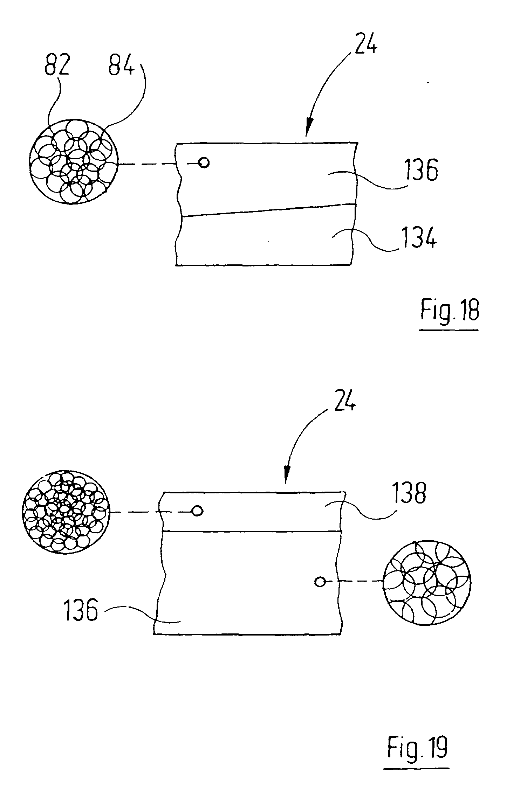

Anstelle von Deflektorteilen, die durchweg aus Skelettschaummaterial bestehen, kann man auch Verbund-Deflektorteile verwenden, wie sie nunmehr unter Bezugnahme auf die Figuren 18 und 19 beschrieben wurden.

Das Deflektorteilmaterial nach Figur 18 hat eine untere Grundschicht 134, die strömungsundurchlässig ist (porenfreies massives Material oder geschlossenporiges Material). Auf der Grundschicht 134 sitzt eine Deflektorschicht 136, die aus einem offenporigen Material besteht, wie oben beschrieben, oder eine Vielzahl diskreter Umlenkelemente (Deflektorrampe, Deflektorgitter) trägt, analog zu dem in Figur 5 gezeigte Deflektionsteil.

Die Grundschicht 134 dient zum einen dazu, die Menge des benötigten Abformmateriales zu verringern. Hierdurch wird zum einen eine Kostenersparnis erzielt, andererseits wird auch die zum Füllen des Formraumes benötigte Zeit herabgesetzt. Schließlich kann man über denjenigen Anteil der Höhe des Formraumes, welcher von der Grundschicht 134 eingenommen wird, auch die Strömungsgeschwindigkeit des Abformmateriales einstellen.

Beim Ausführungsbeispiel nach Figur 19 hat man eine offenporige Grundschicht 138 und eine ebenso offenporige Deflektorschicht 140, welche z.B. durch Skelettschaummaterialien unterschiedlicher (in der Regel oben kleinerer) Porösität gebildet sein können. Auf diese Weise ist es z. B. möglich, das flüssige Abformmaterial in ausreichendem Maße den vorderen Abschnitten des Abformlöffels zuzuführen, trotzdem aber durch eine Vielzahl von den Zähnen benachbarten Flüssigkeits-Streuelementen eine gleichmäßige und starke Umlenkung in zur Bodenwand 16 des Abformlöffels 10 senkrechter Richtung zu erzeugen. Mit einer solchen Verbundstruktur läßt sich das Strömungsmuster im Abformlöffel und die Art und Weise, wie sich der Abformlöffel mit Abformmaterial füllt, einfach vorgeben.

Im einzelnen sind für das Erhalten blasenfreier und präziser Abformungen folgende Parameter von Interesse:

Bezüglich des Abformlöffels selbst: Grundgeometrie des Grundteils, insbesondere Höhe der Umfangswand 14 über der Bodenwand 16; Geometrie und Lage der Verbindungsöffnungen 60; Lage und Verteilung sowie Geometrie der einzelnen Vakuumöffnungen 26; Oberflächenqualität der Innenfläche des Grundteils 12.

Bezüglich des Deflektorteiles: Größe und Lage nicht strömungsdurchlässiger Abschnitte (z.B. Grundschicht 134); Geometrie und Lage von strömungsumlenkenden Bestandteilen; mikroskopischer Aufbau der strömungsumlenkenden Bestandteile; chemische Natur des verwendeten Materiales und sich hieraus ergebende physikalische Eigenschaften, insbesondere Benetzbarkeit durch das Abformmaterial und mechanische Festigkeit sowie mechanische Verformbarkeit; Elastizität; plastische Verformbarkeit (insbesondere bei Metallschäumen), Fixierung des Deflektorteiles am Abformlöffel.

Bezüglich des Abformmateriales: chemische Eigenschaften und sich hieraus ergebende physikalische Eigenschaften, insbesondere rheologische Eigenschaften; Thixotropie; Härtungszeit; gesundheitliche Unbedenklichkeit.

Bezüglich der Anwendung: Vorbehandlung abzuformender Zähne; gegebenenfalls Vorumspritzen besonders problematischer Abdruckbereiche mit dünnflüssigem Abformmaterial; Höhe des an den Abformlöffel angelegten Vakuums; Druck und Strömungsgeschwindigkeit des zugeführten Abformmateriales.

Hierzu wird nun nachstehend im einzelnen Stellung genommen.

### Zu den Eigenschaften des Abdrucklöffels:

Bei den oben beschriebenen Abdrucklöffeln erfolgt das Zuführen des Abformmateriales zu den hintersten Abschnitten der Schenkel der im wesentlichen U-förmigen Formrinne, welche durch die Umfangswand 14, die Bodenwand 16 und die Gaumenwand 18 bzw. die Umfangswand 20 des Abformlöffels 10 begrenzt ist. An das Vakuumanschlußteil 28 wird Vakuum angelegt. Damit erhält man einen Grundfluß des Abformmateriales von distal nach proximal, wobei die Vielzahl vorgesehener Vakuumöffnungen 26 gewährleistet, daß unabhängig von Unsymmetrien im Materialfluß in den beiden Schenkeln des Abformlöffels bis zuletzt Restluft und vom Abformmaterial vor sich hergeschobene Flüssigkeit abgesaugt werden.

Eine zusätzlich vorgesehene Texturierung der Umfangswand 14 und gegebenenfalls der Gaumenwand 18 bzw. der Umfangswand 20 ist nützlich, um zusätzliche bezogen auf die Zahnlängsachse radiale Bewegungskomponenten im Abformmaterial zu erzeugen.

Im einzelnen kann die Texturierung der Innenfläche des Abformlöffels ähnlich aussehen, wie obenstehend unter Bezugnahme auf die Figuren 5 und 6 bzw. 7 und 8 für ein Deflektorteil beschrieben.

Als Material für den Abformlöffel kommt in erster Linie ein Sterilisationsbedingungen standhaltendes Metall oder ein entsprechend standfester Kunststoff in Betracht.

Soweit das Deflektorteil einen für Flüssigkeit undurchlässigen Abschnitt aufweist, läßt sich durch die Randkontur desselben und dessen Höhe die Strömungsgeschwindigkeit des Abformmateriales im Abformlöffel lokal variieren.

### Zu den Deflektormaterialien:

Soweit das Deflektorteil diskrete Deflektorabschnitte wie die Deflektorrampen 88 aufweist, kann es aus einem beliebigen gut formbaren und vorzugsweise plastisch verformbaren Material bestehen, z.B. Kunststoff, Wachs usw.. Die Strömungs-Umlenkeigenschaften bzw. die Steueigenschaften ergeben sich im einzelnen aus der Geometrie der einzelnen Deflektorelemente und deren Anordnung.

Auch rauhe Oberflächen, wie sie z.B. durch Anschneiden eines geschlossenporigen Schaummateriales erhalten werden, können als Streuzentren für das Abformmaterial dienen.

Soweit als Deflektormaterial ein offenporiges Schaummaterial verwendet wird, kommen hierfür nicht nur offenporige Schaummaterialien aus Kunststoff sondern auch solche aus Keramikmaterial, Metall oder anderen anorganischen Materialien in Frage. Der Schaum muß gegen die Strömungsbelastungen, die in ihm beim Durchdrücken des Abformmateriales entstehen, stabil sein, sollte aber keine nennenswert höhere Stabilität aufweisen, so daß er durch Eindrücken eines Zahnes leicht verformt werden kann (Sicherheitsmaßnahme; Verwendbarkeit des Schaummateriales auch für "hohe" Deflektorteile).

Soweit ein ancrganischen Schaummaterial verwendet wird, welches spröde ist, sollte dieses Material vorzugsweise nur als "niederes" Deflektorteil verwendet werden. Bei Verwendung als "hohes" Deflektorteil entstehen durch Eindrücken der Zähne möglicherweise flitterähnliche Fragmente, die die Qualtität des Abdruckes beeinträchtigen. In diesem Falle müßte der Abformlöffel nach einem ersten Einsetzen nochmals dem Mund des Patienten entnommen werden, und man müßte das Mundinnere und das Schaummaterial von losen Fragmenten, z.B. durch Anblasen, befreien.

Für die Wechselwirkung mit dem Abformmaterial sind zum einen die physikalischen Eigenschaften des Deflektormateriales von Interesse, insbesondere die Benetzbarkeit des Materiales durch das Abformmaterial. Soweit das Deflektormaterial nicht von Hause aus die gewünschte Benetzbarkeit (oder nicht Benetzbarkeit) durch das Abformmaterial aufweist, kann es mit einer geeigneten Beschichtung versehen werden.

Eine weitere wichtige Größe der offenporigen Schaummaterialien ist die Porendichte bzw. Porengröße ( = 1/Porendichte) .

Gemäß der praktischen Erprobung der vorliegenden Erfindung werden folgende Polyurethan-Skelettschaummaterialien bevorzugt:

| Materialnummer | Porendichte (Poren pro Inch) | Gerüst Fadendurchmesser |
|---|---|---|
| | | |
| 1 | < = 10 ppi | 0,2 bis 0,4 mm |
| 2 | 10 ppi bis 20 ppi | 0,2 bis 0,3 mm |
| 3 | um 20 ppi | um 0,2 mm |
| 4 | 20 ppi bis 30 ppi | 0,05 bis 0,2 mm |
| 5 | 30 ppi bis 45 ppi | 0,05 bis 0,2 mm |

Die insgesamt erhaltenen Flüssigkeits-Streueigenschaften hängen neben der Porengröße und dem Durchmesser der Skelettzweige auch von der Grundgeometrie des Deflektorteiles ab, insbesondere von dessen Randkontur und Höhe.

In Hinblick darauf, daß das Deflektorteil 24 nicht nur eine Strömungsumlenkung in Richtung auf die Zähne zu vornehmen soll sondern zugleich auch den Fluß des Abformmittels in Richtung zum Vakuumanschlußteil 28 drosseln soll, ist es vorteilhaft, wenn die Dicke des Deflektorteiles 24 zwei Porendurchmesser nicht unterschreitet. Für die Praxis brauchbare Dicken für aus Skelettschaum hergestellte Deflektorteile liegen im Bereich von 2 bis 5 mm für "niedere" Deflektorteile und im Bereich von 4 - 9 mm für "hohe" Deflektorteile. Dem Zahnarzt werden solche vorgefertigten Deflektorteile mit unterschiedlicher Porendichte und unterschiedlicher Höhe zur Verfügung gestellt, damit er hieraus das im Hinblick auf das jeweilige Gebiß richtige "niedere" oder "hohe" Material heraussuchen kann. Gegebenenfalls wird der Anwender unterschiedliche Deflektorteile an unterschiedliche Stellen im Abdruck einsetzen oder mehrere Deflektorteile übereinander verwenden (wenigstens partiell).

### Für das Abformmaterial gilt folgendes:

Im Prinzip sind alle gängigen Abformmaterialien geeignet, also insbesondere Hydrokolloide, Alginate, Polyäther, Kunststoffe, Gipse, kondensationsvernetzende Silikone, additionsvernetzende Silikone usw.. Hiervon werden additionsvernetzende Silikone bevorzugt.

Viele üblicherweise verwendeten Abformmaterialien haben thixotrope Eigenschaft. Für die Abformung werden im Hinblick auf gutes Fließen erfindungsgemäß nicht oder nur wenig thixotrope Abformmaterialien bevorzugt.

Verwendet man z.B. ein PUR-Skelettschaummaterial so erfolgt die Abstimmung zwischen der Porendichte (inverse Porengröße) des Schaummateriales und der Viskosität des Abformmateriales z.B. in etwa gemäß nachfolgender Tabelle:

| Porendichte (ppi) | Viskosität (Pasec) |
|---|---|
| | |
| < 10 | 150 - 350 |
| 10 - 20 | 30 - 300 |
| 20 ± e | 7,5 - 50 |
| 20 - 30 | 5 - 30 |
| 30 - 45 | 1 - 10 |
| (e = kleine Zahl zwischen 0 und etwa 2) | |

Bezüglich des Einbringens des Abformmateriales ist folgendes zu berücksichtigen:

Die Geschwindigkeit, mit welcher das Abformmaterial in den Abformlöffel eingespeist ist, muß so groß sein, daß noch keine Aushärtung eingetreten ist, bis die Materialfront das Vakuumanschlußteil 28 erreicht hat. Andererseits sollte die Zuführung so langsam erfolgen, daß das Abformmateial ausreichend Zeit hat, auch enge und hinterschnittene Bereiche des Kiefers auszufüllen.

Als Grundwert für die Zuführgeschwindigkeit des Abformmateriales kann eine Zeitspanne von 20 sec bis 40 sec, bevorzugt 30 sec für das vollständige Leeren einer handelsüblichen Doppelkartusche für etwa 50 ml Gesamtabformmaterialvolumen dienen. Dieser Grundwert kann dann je nach den speziell angefundenen Bedingungen und je nach dem speziell verwendeten Deflektorteil nach unten bzw. oben abgewandelt werden.

Der an den Abformlöffel beim Füllen mit Abformmaterial angelegte Unterdruckwert (relative Druckabsenkung gegenüber der Umgebung) kann in der Praxis zwischen 100 und 350 Millibar betragen. Werte um 150 Millibar werden bevorzugt verwendet.

Falls gewünscht, können besonders kritische Teilbereiche des abzuformenden Kiefers vor dem Positionieren des Abformlöffels mit einem dünnflüssigen Abformmaterial manuell umspritzt werden, welches zu dem Abformlöffel über das Speiseteil 40 zugeführten Haupt-Abformmaterial chemisch ähnlich ist, so daß sich beide Abformmaterialien miteinander verbinden können. Das Umspritz-Abformmaterial wird mit solcher Härtungszeit gewählt, daß es noch flüssig ist, wenn das Haupt-Abformmaterial die Zähne erreicht. Auf diese Weise kann das Haupt-Abformmaterial das Umspritz-Abformmaterial noch in Zahntaschen oder klein bemessene Kavitäten hineindrücken, so daß man von dem besonders interessierenden Bereichen ein blasenfreies genaues Abbild erhält.

Geht man wie oben beschrieben zum Herstellen einer Abformung vor, so sind Fehler ausgeräumt, wie sie beim herkömmlichen Herstellen von Abformungen auftreten, insbesondere Verzug, eingeschlossene Luftblasen, nicht scharfe Abbildung von Präparationsrändern, ungenaue Abbildung der Präparationsgrenzen bzw. subgingival gelegener Abschnitte von Zahnwurzeloberflächen, nicht zufriedenstellende Wiedergabe von tiefen Kavitäteneinsschnitten.

Obenstehend wurde ein Abformteil und seine Anwendung für die Herstellung von Zahnabdrücken beschrieben. Von der Eigenschaft des Abformlöffels, ein visköses Material auch schlecht zugänglichen Stellen eines Kiefers zuzuführen, kann man auch zu Therapiezwecken Gebrauch machen: Man baut in ein Grundmaterial, dessen rheologische Eigenschaften eines Abformateriales gleich- oder nahekommen oder das ein Abformmaterial ist, medizinische Wirkstoffe ein. Dies können heilende Wirkstoffe, insbesondere entzündungshemmende und Entzündungen vorbeugende Wirkstoffe, desinfinzierende Wirkstoffe, Antiadhäsiva, Anästhetika, die mechanischen und/oder chemischen Oberflächeneigenschaften der Zahnhartgewebe beeinflussende Wirkstoffe oder Mischungen der vorgenannten Wirkstoffe sein.

Durch Steuerung der Benetzungsfähigkeit der Zähne oder des diese umgebenden Weichgewebes (hydrophobes oder hydrophiles Therapiematerial) kann die Abgabe des darin enthaltenen Wirkstoffes kontrolliert und gezielt beeinflußt werden.

## Patentansprüche

1. Dentaler Abformlöffel mit mindestens einer Umfangswand (14, 18; 14, 20) und einer Bodenwand (16), wobei diese Wände eine Formrinne (21) begrenzen, welche zumindest einen Teil einer Zahnreihe (106) aufnehmen kann und mit einem Speisestutzen (48) für Abformmaterial sowie einem Anschlußstutzen (38) zur Entlüftung in Verbindung steht, **dadurch gekennzeichnet, daß** in der Formrinne eine Vielzahl von Deflektorelementen (82; 88; 96) angeordnet ist.

2. Abformlöffel nach Anspruch 1, **dadurch gekennzeichnet, daß** zumindest ein Teil der Deflektorelemente (82; 88; 96) von einer Umfangswand (14, 20) oder der Bodenwand (16) getragen ist.

3. Abformlöffel nach Anspruch 1 oder 2, **dadurch gekennzeichnet, daß** zumindest ein Teil der Deflektorelemente (82; 88; 96) auf einem in die Formrinne (21) eingesetzten Deflektionsteil (24) angeordnet ist.

4. Abformlöffel nach Anspruch 3, **dadurch gekennzeichnet, daß** diskrete Deflektorelemente (88; 96) von einer der Bodenwand (16) benachbarten Basiswand (86; 94) des Deflektionsteiles (24) getragen sind.

5. Abformlöffel nach Anspruch 4, **dadurch gekennzeichnet, daß** die Deflektorelemente (88; 96) in aufeinanderfolgenden Reihen äquidistant angeordnet sind.

6. Abformlöffel nach Anspruch 5, **dadurch gekennzeichnet, daß** die Deflektorelemente (88) aufeinanderfolgender Reihen um eine halbe Teilung gegeneinander versetzt sind.

7. Abformlöffel nach einem der Ansprüche 1 bis 6, **dadurch gekennzeichnet, daß** die Deflektorelemente (96) aus Wachs oder einem plastisch verformbaren Kunststoff oder Metall hergestellt sind.

8. Abformlöffel nach einem der Ansprüche 1 bis 7, **dadurch gekennzeichnet, daß** die Deflektorelemente (88; 96) eine zur Bodenwand (16) geneigte Deflektionsfläche aufweisen.

9. Abformlöffel nach einem der Ansprüche 1 bis 8, **dadurch gekennzeichnet, daß** die Deflektorelemente (96) für Flüssigkeit teildurchlässig sind.

10. Abformlöffel nach Anspruch 1 oder 2, **dadurch gekennzeichnet, daß** die Deflektorelemente durch Wände oder Zweige (82) eines offenporigen Schaummateriales gebildet sind, welches ein zusammenhängendes Deflektionsteil (24) bildet.

11. Abformlöffel nach Anspruch 10, **dadurch gekennzeichnet, daß** die Zweige (82) draht- oder fadenähnlich sind (Skelettschaummaterial).

12. Abformlöffel nach Anspruch 10 oder 11, **dadurch gekennzeichnet, daß** das offenporige Schaummaterial eine Porendichte von mindestens 10 ppi aufweist.

13. Abformlöffel nach Anspruch 12, **dadurch gekennzeichnet, daß** die Porendichte des offenporigen Schaummateriales zwischen 20 und 45 ppi beträgt.

14. Abformlöffel nach einem der Ansprüche 12 oder 13, **dadurch gekennzeichnet, daß** der Durchmesser der draht- oder fadenähnlichen Zweige des Skelettschaummateriales 1/30 bis 1/5 der Porengröße bzw. Maschenweite beträgt.

15. Abformlöffel nach Anspruch 14, **dadurch gekennzeichnet, daß** das Verhältnis zwischen dem Durchmesser der Zweige des Skelettschaummateriales und dessen Poren- bzw. Maschengröße etwa 1:10 beträgt.

16. Abformlöffel nach einem der Ansprüche 10 bis 15, **dadurch gekennzeichnet, daß** das offenporige Schaummaterial ein Kunststoff-, insbesondere PUR-Skelettschaummaterial ist.

17. Abformlöffel nach einem der Ansprüche 10 bis 15, **dadurch gekennzeichnet, daß** das Schaummaterial ein anorganisches, insbesondere metallisches oder keramisches Schaummaterial ist.

18. Abformlöffel nach einem der Ansprüche 10 bis 17, **dadurch gekennzeichnet, daß** das Schaummaterial aus Plattenmaterial hergestellt ist.

19. Abformlöffel nach einem der Ansprüche 11 bis 18, **dadurch gekennzeichnet, daß** das Schaummaterial (136; 140) von einer Grundschicht (134; 138) getragen ist.

20. Abformlöffel nach Anspruch 19, **dadurch gekennzeichnet, daß** die Grundschicht (134) für Flüssigkeiten undurchlässig ist.

21. Abformlöffel nach Anspruch 20, **dadurch gekennzeichnet, daß** die Grundschicht (138) durch ein offenporiges Schaummaterial gebildet ist.

22. Abformlöffel nach Anspruch 21, **dadurch gekennzeichnet, daß** die Grundschicht (138) größere Poren aufweist, als das von ihr getragene Schaummaterial (136).

23. Abformlöffel nach einem der Ansprüche 10 bis 22, **dadurch gekennzeichnet, daß** das Schaummaterial mit einem Dichtabschnitt (122) versehen ist, der über die Umfangswand (14) hinweggeführt ist und eine für Flüssigkeit undurchlässige Haut (124) trägt.

24. Abformlöffel nach einem der Ansprüche 3 bis 23, **dadurch gekennzeichnet, daß** die Bodenwand (16) und/oder zumindest eine der Umfangswände (14, 20) mechanisch wirkende Befestigungsmittel (22) aufweist, die mit dem Deflektionsteil (24) zusammenarbeiten.

25. Abformlöffel nach einem der Ansprüche 3 bis 24, **dadurch gekennzeichnet, daß** das Deflektionsteil (24) so nieder ist, daß es bei appliziertem Abformlöffel (10) die Zähne (106) nicht berührt und die Deflektorelemente (88; 96) aus einem durch das Abformmaterial nicht benetzbaren Material besteht oder mit einem solchen beschichtet ist.

26. Abformlöffel nach einem der Ansprüche 3 bis 24, **dadurch gekennzeichnet, daß** das Deflektionsteil (24) so hoch ist, daß es bei appliziertem Abformlöffel zumindest einen der Zähne berührt und daß die Deflektionselemente aus einem das Abformmaterial benetzendem Material bestehen oder mit einem solchen beschichtet sind.

27. Abformmaterial nach einem der Ansprüche 1 bis 26, **dadurch gekennzeichnet, daß** die Unterseite der Bodenwand (16) ein Speiseteil (40) mit im wesentlichen gleicher Randkontur trägt, welches einen Abformmaterial-Speisestutzen (48) mit Verbindungsöffnungen (60) strömungsmäßig verbindet, welche im hinteren Abschnitt der Bodenwand (16) vorgesehen sind und welche vorzugsweise bei ihrem hinteren Ende eine in Strömungsrichtung des Abformmateriales schräg angestellten Rand (62) aufweisen.

28. Abformlöffel nach Anspruch 27, **dadurch gekennzeichnet, daß** das Speiseteil (40) über mindestens eine Rastverbindung (64-70) mit dem durch die Bodenwand (16) und die Umfangswände (14, 18; 14, 20) gebildeten Grundteil (12) verbunden ist.

29. Abformlöffel nach einem der Ansprüche 1 bis 28, **dadurch gekennzeichnet, daß** die Umfangswand (14) in ihrem vorderen Abschnitt eine Vielzahl von Vakuumöffnungen (26) aufweist, die mit einem Vakuumanschlußteil (28) in Verbindung stehen.

30. Abformlöffel nach Anspruch 29 **dadurch gekennzeichnet, daß** die Vakuumöffnungen (26) in einem recheckige Randkontur aufweisenden Abschnitt der Umfangswand (14) regelmäßig verteilt sind.

31. Abformlöffel nach Anspruch 29 oder 30, **dadurch gekennzeichnet, daß** das Vakuumanschlußteil (28) durch mindestens eine Rastverbindung (34, 36) lösbar mit den durch Bodenwand (16) und Umfangswände (14, 18; 14, 20) gebildeten Grundteil (12) verbunden ist, vorzugsweise unter Zwischenschaltung einer Dichtung (30).

32. Abformlöffel nach einem der Ansprüche 1 bis 32, **dadurch gekennzeichnet, daß** die Umfangswand (16) mindestens ein Dichteil (72) trägt.

33. Abformlöffel nach Anspruch 32, **dadurch gekennzeichnet, daß** zumindest ein Abschnitt des Dichtteiles (72) mindestens eine Dichtlippe (78, 80) trägt.

34. Abformlöffel nach Anspruch 33, **dadurch gekennzeichnet, daß** der genannte Abschnitt des Dichtteiles (72) zwei unter unterschiedlichen Winkeln angestellte Dichtlippen (78, 80) trägt.

35. Abformlöffel nach einem der Ansprüche 1 bis 34, **dadurch gekennzeichnet, daß** die Viskosität eines mit ihm verwendeten Abformmateriales im Hinblick auf die Größe der Deflektorelemente (82; 88; 96) so eingestellt ist, daß der zwischen Abformlöffel (10) und Kiefer des Patienten eingeschlossene Formraum vom Boden des Abformlöffels (10) zu den Zähnen hin gefüllt wird, so daß Einschlüsse von Luft und Flüssigkeiten vermieden werden.

36. Abformlöffel nach Anspruch 35, **dadurch gekennzeichnet, daß** die Größe der Deflektorelemente (82; 88; 96) und die Viskosität des Abformmateriales für kleine Strömungsgeschwindigkeiten wie folgt einander zugeordnet sind:
| Porendichte (ppi) | Viskosität (Pasec) |
|---|---|
| | |
| < 10 | 150 - 350 |
| 10 - 20 | 30 - 300 |
| 20 ± e | 7, 5 - 50 |
| 20 - 30 | 5 - 30 |
| 30 - 45 | 1 - 10 |
| (e = kleine Zahl zwischen 0 und etwa 2) | |

37. Abformlöffel nach Anspruch 35, **dadurch gekennzeichnet, daß** es nicht oder nur wenig thixotrop ist.

38. Abformlöffel nach einem der Ansprüche 1 bis 34, **dadurch gekennzeichnet, daß** die Viskosität eines mit ihm verwendeten Thrapiemateriales im Hinblick auf die Größe der Deflektorelemente (82; 88; 96) so eingestellt ist, daß der zwischen Abformlöffel (10) und Kiefer des Patienten eingeschlossene Formraum vom Boden des Abformlöffels (10) zu den Zähnen hin gefüllt wird, so daß Einschlüsse von Luft und Flüssigkeiten vermieden werden.

39. Abformlöffel nach Anspruch 38, daß das Therapiematerial eine oder mehrere aus der nachstehenden Gruppe von Therapiesubstanzen enthält: heilende Wirkstoffe, insbesondere entzündungshemmende und Entzündungen vorbeugende Wirkstoffe, desinfizierende Wirkstoffe, Antiadhäsiva, Anästhetika, die mechanischen und/oder chemischen Oberflächeneigenschaften der Zahnhartgewebe beeinflussende Wirkstoffe oder Mischungen der vorgenannten Wirkstoffe.

## Claims

1. Dental moulding spoon with at least one peripheral wall (14, 18; 14, 20) and a base wall (16), where these walls delimit a moulding channel (21) which can hold at least part of a row of teeth (106) and which is connected with a feed nozzle (48) for moulding material and a connecting nozzle (38) for air evacuation, **characterised in that** in the moulding channel is arranged a multiplicity of deflector elements (82; 88; 96).

2. Moulding spoon according to Claim 1, **characterised in that** at least some of the deflector elements (82; 88; 96) are supported by a peripheral wall (14, 20) or the base wall (16).

3. Moulding spoon according to Claim 1 or 2, **characterised in that** at least some of the deflector elements (82; 88; 96) are arranged on a deflection part (24) inserted in the moulding channel (21).

4. Moulding spoon according to Claim 3, **characterised in that** discrete deflector elements (88; 96) are carried by a base wall (86; 94) of the deflection part (24) adjacent to the base wall (16).

5. Moulding spoon according to Claim 4, **characterised in that** the deflector elements (88; 96) are arranged equidistant in successive rows.

6. Moulding spoon according to Claim 5, **characterised in that** the deflector elements (88) of successive rows are offset to each other by half their pitch.

7. Moulding spoon according to any of Claims 1 to 6, **characterised in that** the deflector elements (96) are made of wax or a plastically deformable plastic or metal.

8. Moulding spoon according to any of Claims 1 to 7, **characterised in that** the deflector elements (88; 96) have a deflection surface angled in relation to the base wall (16).

9. Moulding spoon according to any of Claims 1 to 8, **characterised in that** the deflector elements (96) are partly permeable to fluid.

10. Moulding spoon according to Claim 1 to 2, **characterised in that** the deflector elements are formed by walls or branches (82) of an open-pore foam material which forms a cohesive deflection part (24).

11. Moulding spoon according to Claim 10, **characterised in that** the branches (82) are wire- or thread-like (skeleton foam material).

12. Moulding spoon according to Claim 10 or 11, **characterised in that** the open-pore foam material has a pore density of at least 10 ppi.

13. Moulding spoon according to Claim 12, **characterised in that** the pore density of the open-pore foam material is between 20 and 45 ppi.

14. Moulding spoon according to Claim 12 or 13, **characterised in that** the diameter of the wire- or thread-like branches of the skeleton foam material amounts to 1/30 to 1/5 of the pore density or mesh width.

15. Moulding spoon according to Claim 14, **characterised in that** the ratio between the diameter of the branches of the skeleton foam material and its pore or mesh size is around 1:10.

16. Moulding spoon according to any of Claims 10 to 15, **characterised in that** the open-pore foam material is a plastic, in particular a PUR skeleton foam material.

17. Moulding spoon according to any of Claims 10 to 15, **characterised in that** the foam material is an inorganic, in particular metallic or ceramic foam material.

18. Moulding spoon according to any of Claims 10 to 17, **characterised in that** the foam material is made from plate material.

19. Moulding spoon according to any of Claims 11 to 18, **characterised in that** the foam material (136; 140) is carried by a base layer (134; 138).

20. Moulding spoon according to Claim 19, **characterised in that** the base layer (134) is impermeable to fluids.

21. Moulding spoon according to Claim 20, **characterised in that** the base layer (138) is formed by an open-pore foam material.

22. Moulding spoon according to Claim 21, **characterised in that** the base layer (138) has larger pores than the foam material (136) it carries.

23. Moulding spoon according to any of Claims 10 to 22, **characterised in that** the foam material has a sealed section (122) which is guided over the peripheral wall (14) and carries a skin (124) impermeable to fluid.

24. Moulding spoon according to any of Claims 3 to 23, **characterised in that** the base wall (16) and/or at least one of the peripheral walls (14, 20) has mechanically active fixing means (22) which co-operate with the deflection part (24).

25. Moulding spoon according to any of Claims 3 to 24, **characterised in that** the deflection part (24) is so low that when the moulding spoon (10) is applied, said part does not touch the teeth (106) and the deflector elements (88; 96) comprise or are coated with a material which cannot be wetted by the moulding material.

26. Moulding spoon according to any of Claims 3 to 24, **characterised in that** the deflection part (24) is so high that when the moulding spoon is applied, said part touches at least one of the teeth and that the deflector elements comprise or are coated with a material wetting the moulding material.

27. Moulding spoon according to any of Claims 1 to 26, **characterised in that** the underside of the base wall (16) carries a feed part (40) with substantially even peripheral contour which hydrodynamically combines a moulding material feed nozzle (48) with connecting openings (60) that are provided in the rear section of the base wall (16) and preferably have at their rear end an edge (62) placed obliquely in the flow direction of the moulding material.

28. Moulding spoon according to Claim 27, **characterised in that** the feed part (40) is connected via at least one latch connection (64 - 70) with the base part (12) formed by the base wall (16) and the peripheral walls (14, 18; 14, 20).

29. Moulding spoon according to any of Claims 1 to 28, **characterised in that** the peripheral wall (14) in its front section has a multiplicity of vacuum openings (26) which are connected with a vacuum connection part (28).

30. Moulding spoon according to Claim 29, **characterised in that** the vacuum openings (26) are evenly distributed in a section of the peripheral wall (14) having a rectangular edge contour.

31. Moulding spoon according to Claim 29 to 30, **characterised in that** the vacuum connection part (28) is detachably connected by at least one latch connection (34, 36) with the base part (12) formed by the base wall (16) and peripheral walls (14, 18; 14, 20), preferably with a seal (30) in between.

32. Moulding spoon according to any of Claims 1 to 31, **characterised in that** the peripheral wall (16) carries at least one sealing part (72).

33. Moulding spoon according to Claim 32, **characterised in that** at least one section of the sealing part (72) carries at least one sealing lip (78, 80).

34. Moulding spoon according to Claim 33, **characterised in that** said section of the sealing part (72) carries two sealing lips (78, 80) set at different angles.

35. Moulding spoon according to any of Claims 1 to 34, **characterised in that** the viscosity of a moulding material used with said spoon is set in relation to the size of the deflector elements (82; 88; 96) such that the cavity enclosed between the moulding spoon (10) and the patient's jaw is filled from the base of the moulding spoon (10) to the teeth so that inclusions of air and fluid can be avoided.

36. Moulding spoon according to Claim 35, **characterised in that** the size of the deflector elements (82; 88; 96) and the viscosity of the moulding material for small flow speeds are allocated to each other as follows:
| Pore density (ppi) | Viscosity (Pasec) |
|---|---|
| < 10 | 150 - 350 |
| 10 - 20 | 30 - 300 |
| 20 ± e | 7.5 - 50 |
| 20 - 30 | 5 - 30 |
| 30 - 45 | 1 - 10 |
| (e = small number between 0 and around 2). | |

37. Moulding spoon according to Claim 35, **characterised in that** it is not or only slightly thixotropic.

38. Moulding spoon according to any of Claims 1 to 34, **characterised in that** the viscosity of a treatment material used with said spoon is set in relation to the size of the deflector elements (82; 88; 96) such that the cavity enclosed between the moulding spoon (10) and the patient's jaw is filled from the base of the moulding spoon (10) to the teeth so that inclusions of air and fluid can be avoided.

39. Moulding spoon according to Claim 38, **characterised in that** the treatment material contains one or more of the following groups of treatment substances: healing agents, in particular inflammation-inhibiting and inflammation-preventing agents, disinfectant substances, anti-adhesives, anaesthetics, substances influencing the mechanical and/or chemical surface properties of the tooth core tissue, or mixtures of said substances.

## Revendications

1. Porte-empreinte dentaire comprenant au moins une paroi circonférentielle (14, 18 ; 14, 20) et une paroi inférieure (16), ces parois définissant une gouttière de moulage (21) qui peut accueillir au moins une partie d'une rangée de dents (106) et qui est reliée à une tubulure d'alimentation (48) pour le matériau à empreinte, ainsi qu'à une tubulure de raccordement (38) pour l'évacuation de l'air, **caractérisé en ce qu'**une pluralité d'éléments déflecteurs (82 ; 88 ; 96) sont disposés dans la gouttière de moulage.

2. Porte-empreinte selon la revendication 1, **caractérisé en ce qu'**au moins une partie des éléments déflecteurs (82 ; 88 ; 96) est supportée par une paroi circonférentielle (14, 20) ou la paroi inférieure (16).

3. Porte-empreinte selon la revendication 1 ou 2, **caractérisé en ce qu'**au moins une partie des éléments déflecteurs (82 ; 88 ; 96) est disposée sur une pièce de déflection (24) insérée dans la gouttière de moulage (21 ).

4. Porte-empreinte selon la revendication 3, **caractérisé en ce que** des éléments déflecteurs discrets ( 88; 96) sont supportés par une paroi de base (86 ; 94) de la pièce de déflection (24) voisine de la paroi inférieure (16).

5. Porte-empreinte selon la revendication 4, **caractérisé en ce que** les éléments déflecteurs (88 ; 96) sont disposés en rangées successives équidistantes les unes par rapport aux autres.

6. Porte-empreinte selon la revendication 5, **caractérisé en ce que** les éléments déflecteurs (88) des rangées successives sont décalés entre eux en quinconce d'une demi subdivision.

7. Porte-empreinte selon l'une des revendications 1 à 6, **caractérisé en ce que** les éléments déflecteurs (96) sont en cire ou en métal, ou fabriqués dans une matière synthétique plastiquement déformable.

8. Porte-empreinte selon l'une des revendications 1 à 7, **caractérisé en ce que** les éléments déflecteurs (88 ; 96) présentent une surface de déflection inclinée vers la paroi inférieure (16).

9. Porte-empreinte selon l'une des revendications 1 à 8, **caractérisé en ce que** les éléments déflecteurs (96) sont partiellement perméables aux liquides.

10. Porte-empreinte selon la revendication 1 ou 2, **caractérisé en ce que** les éléments déflecteurs sont formés par les parois ou les ramifications (82) d'un matériau alvéolaire ou mousse à pores ouverts qui forme une pièce de déflection d'un seul tenant (24).

11. Porte-empreinte selon la revendication 10, **caractérisé en ce que** les ramifications (82) ressemblent à des fils ou à des brins (structure en matériau alvéolaire).

12. Porte-empreinte selon la revendication 10 ou 11, **caractérisé en ce que** le matériau alvéolaire à pores ouverts présente une densité de pores d'au moins 10 ppi.

13. Porte-empreinte selon la revendication 12, **caractérisé en ce que** la densité de pores du matériau alvéolaire à pores ouverts est comprise entre 20 et 45 ppi.

14. Porte-empreinte selon l'une des revendications 12 ou 13, **caractérisé en ce que** le diamètre des ramifications de la structure de squelette en matériau alvéolaire, de type fil ou brin, correspond à 1/30 à 1/5 de la grosseur de pore ou encore de la largeur de maille.

15. Porte-empreinte selon la revendication 14, **caractérisé en ce que** le rapport entre le diamètre des ramifications de la structure de squelette en matériau alvéolaire et sa grosseur de pore ou encore sa largeur de maille est de l'ordre de 1:10.

16. Porte-empreinte selon l'une des revendications 10 à 15, **caractérisé en ce que** le matériau alvéolaire à pores ouverts est une matière synthétique, en particulier un matériau de structure de squelette alvéolaire à base de polyuréthane.

17. Porte-empreinte selon l'une des revendications 10 à 15, **caractérisé en ce que** le matériau alvéolaire de squelette est un matériau alvéolaire inorganique, en particulier un matériau alvéolaire métallique ou céramique.

18. Porte-empreinte selon l'une des revendications 10 à 17, **caractérisé en ce que** le matériau alvéolaire est fabriqué à partir d'un matériau stratifié en feuilles.

19. Porte-empreinte selon l'une des revendications 11 à 18, **caractérisé en ce que** le matériau alvéolaire (136 ; 140) est porté par une couche de base (134 ; 138).

20. Porte-empreinte selon la revendication 19, **caractérisé en ce que** la couche de base (134) est imperméable aux liquides.

21. Porte-empreinte selon la revendication 20, **caractérisé en ce que** la couche de base (138) est constituée d'un matériau alvéolaire ou mousse à pores ouverts.

22. Porte-empreinte selon la revendication 21, **caractérisé en ce que** la couche de base (138) présente des pores plus gros que ceux du matériau alvéolaire qu'elle porte (136).

23. Porte-empreinte selon l'une des revendications 10 à 22, **caractérisé en ce que** le matériau alvéolaire est pourvu d'un segment étanche (122), qui s'étend au-delà de la paroi circonférentielle (14) et qui est pourvu d'une pellicule imperméable aux liquides (124).

24. Porte-empreinte selon l'une des revendications 3 à 23, **caractérisé en ce que** la paroi inférieure (16) et/ou au moins l'une des parois circonférentielles (14, 20) comporte des moyens de fixation agissant mécaniquement (22), qui coopèrent avec la pièce de déflection (24).

25. Porte-empreinte selon l'une des revendications 3 à 24, **caractérisé en ce que** la pièce de déflection (24) est si basse, que lorsque le porte-empreinte (10) est appliqué, il ne touche pas les dents (106) et **en ce que** les éléments déflecteurs (88 ; 96) sont constitués d'un matériau ne pouvant être mouillé par le matériau à empreinte ou qu'ils sont recouverts d'un matériau de ce type.

26. Porte-empreinte selon l'une des revendications 3 à 24, **caractérisé en ce que** la pièce de déflection (24) est si haute, que lorsque le porte-empreinte (10) est appliqué, il touche au moins l'une des dents et **en ce que** les éléments déflecteurs sont constitués d'un matériau mouillant le matériau à empreinte ou qu'ils sont recouverts d'un matériau de ce type.

27. Porte-empreinte selon l'une des revendications 1 à 26, **caractérisé en ce que** le dessous de la paroi inférieure (16) porte un élément d'alimentation (40) présentant un contour de bord sensiblement identique, qui relie sur le plan de l'écoulement une tubulure d'alimentation en matériau à empreinte (48) à des ouvertures de raccordement (60), qui sont prévues dans la partie arrière de la paroi inférieure (16) et qui comportent de préférence en leur extrémité arrière un bord (62) orienté obliquement dans le sens de l'écoulement du matériau à empreinte.

28. Porte-empreinte selon la revendication 27, **caractérisé en ce que** l'élément d'alimentation (40) est relié à l'élément de base (12) formé par la paroi inférieure (16) et les parois circonférentielles (14, 18 ; 14, 20) par au moins un raccord encliquetable (64-70).

29. Porte-empreinte selon l'une des revendications 1 à 28, **caractérisé en ce que** la paroi circonférentielle (14), dans sa partie avant, comporte une pluralité d'ouvertures pour le vide (26), qui sont reliées à un élément de raccordement au vide (28).

30. Porte-empreinte selon la revendication 29, **caractérisé en ce que** les ouvertures pour le vide (26) sont régulièrement réparties dans un segment de la paroi circonférentielle (14) présentant un pourtour de forme rectangulaire.

31. Porte-empreinte selon la revendication 29 ou 30, **caractérisé en ce que** l'élément de raccordement au vide (28) est relié de façon amovible à l'élément de base (12) formé par la paroi inférieure (16) et les parois circonférentielles (14, 18 ; 14, 20) par au moins un raccord encliquetable (34, 36), en intercalant de préférence une garniture d'étanchéité (30).

32. Porte-empreinte selon l'une des revendications 1 à 31, **caractérisé en ce que** la paroi circonférentielle (14) comporte au moins un élément d'étanchéité (72).

33. Porte-empreinte selon la revendication 32, **caractérisé en ce qu'**au moins un segment de l'élément d'étanchéité (72) comporte au moins une lèvre d'étanchéité (78, 80).

34. Porte-empreinte selon la revendication 33, **caractérisé en ce que** le segment cité de l'élément d'étanchéité (72) comporte deux lèvres d'étanchéité (78, 80) orientées selon des angles différents.

35. Porte-empreinte selon l'une des revendications 1 à 34, **caractérisé en ce que** la viscosité d'un matériau à empreinte conjointement utilisé est ajustée en fonction de la taille des éléments déflecteurs (82 ; 88 ; 96) de telle sorte que l'espace de moulage englobé entre le porte-empreinte (10) et la mâchoire du patient est rempli, depuis le fond du porte-empreinte (10) jusqu'aux dents, si bien que des vides d'air ou des inclusions de liquide sont empêchés.

36. Porte-empreinte selon la revendication 35, **caractérisé en ce que** le rapport entre la taille des éléments déflecteurs (82 ; 88 ; 96) et la viscosité du matériau à empreinte pour de faibles vitesses d'écoulement sont liés entre eux comme suit :
| Densité des pores en pores par inch (ppi) | Viscosité (PaS) |
|---|---|
| inférieure à 10 | 150 à 350 |
| entre 10 et 20 | 30 à 300 |
| 20 ± e | 7,5 à 50 |
| entre 20 et 30 | 5 à 30 |
| entre 3 0 et 45 | 1 à 10 |
| (e = petit nombre entre 0 et environ 2) | |

37. Porte-empreinte selon la revendication 35, **caractérisé en ce qu'**il est peu ou pas thixotrope.

38. Porte-empreinte selon l'une des revendications 1 à 34, **caractérisé en ce que** la viscosité d'un matériau thérapeutique utilisé conjointement est ajustée en fonction de la taille des éléments déflecteurs (82 ; 88 ; 96) de telle sorte que l'espace de moulage englobé entre le porte-empreinte (10) et la mâchoire du patient est rempli, depuis le fond du porte-empreinte (10) jusqu'aux dents, si bien que des vides d'air ou des inclusions de liquide sont empêchés.

39. Porte-empreinte selon la revendication 38, **caractérisé en ce que** le matériau thérapeutique contient une ou plusieurs substances thérapeutiques choisies parmi les groupes des principes actifs curatifs, en particulier des principes actifs freinant, entravant ou prévenant les inflammations, des principes actifs désinfectants, des anti-adhésifs, des anesthésiques, des principes actifs ayant une action sur les propriétés superficielles mécaniques et/ou chimiques du tissu dentaire dur, ou des mélanges des principes actifs précités.
